# EUROPEAN PATENT APPLICATION

(11) **EP 2 865 685 A1**
(43) Date of publication of application: **29.04.2015**
(21) Application number: 14189607.6
(22) Date of filing: 21.10.2014
(51) Int. Cl.: C07K 1/26

(54) **Electrophoretic analysis of a sample using N-Lauroylsarcosine**

(30) Priority: 24.10.2013 EP 13190095
(71) Applicant: Westfälische Wilhelms-Universität Münster, 48149 Münster (DE)
(72) Inventor: Bernick, Friedrich, 48149 Muenster (DE); Schnekenburger, Juergen, 48149 Muenster (DE); Ossig, Rainer, 48149 Muenster (DE)
(74) Representative: Schiweck, Weinzierl & Koch

(57) **Abstract**

Provided is a method of carrying out electrophoresis on samples that contain a guanidinium salt, a potassium salt and/or a chaotropic agent. In the method the pH of the sample is adjusting to a value of 8.0 or higher, and N-Lauroylsarcosine and a reducing agent are added. The buffer components in a gel for a tris/glycine electrophoresis system are adjusted in the method to higher concentrations. Gel electrophoresis may be carried out with a cathode buffer that contains N-Lauroylsarcosine.

## Description

### FIELD OF THE INVENTION

The present invention relates to electrophoretic analysis of a sample, which contains a chaotropic agent, a guanidinium salt, and/or a potassium salt. Disclosed is in particular a gel electrophoresis method, such as a polyacrylamide gel electrophoresis (PAGE) method or an agarose electrophoresis method, for direct analysis of a sample, where the sample includes a chaotropic agent, a guanidinium salt, and/or a potassium salt. Provided are also an electrophoresis sample buffer, an electrophoresis gel buffer, an electrophoresis running buffer, and a kit for direct analysis by electrophoresis of a protein sample that contains a chaotropic agent, a guanidinium salt, and/or a potassium salt.

### BACKGROUND OF THE INVENTION

The following discussion of the background of the invention is merely provided to aid the reader in understanding the invention and is not admitted to describe or constitute prior art to the present invention.

Electrophoretic analysis, most frequently performed as gel electrophoresis, typically polyacrylamide gel electrophoresis (PAGE), is the most widely used analytical method to resolve peptides and proteins of a sample that is suspected to include peptides and/or proteins. An electrophoretic separation is also the standard technique used to assess the purity of a protein. This technique is also widely-used for the estimation of molecular weights of peptides and proteins and standard practice for the analytical as well as preparative separation of individual components of mixtures of proteins and peptides. In gel electrophoretic analysis, the molecules of interest are separated into bands according to the rate at which an imposed electric field causes them to migrate through a polymer gel. For the application of gel electrophoresis it is an essential prerequisite that the proteins and peptides to be analyzed are entirely dissolved in a suitable sample buffer.

Chaotropic agents such as guanidinium hydrochloride are frequently used for solvation and denaturation of proteins which are difficult to dissolve, including, for example, hydrophobic peptides, proteins, protein precipitates and protein complexes from cells or tissues. Guanidinium hydrochloride or guanidine hydrochloride (GuHCl; CH₆ClN₃; CAS number 50-01-1) is one of the strongest denaturants used in biochemical studies (see e.g. Arakawa, T, and Timasheff, SN, Biochemistry [1984] 23, 5924-5929). In particular, upon recombinant expression of proteins, e.g. in bacteria such as *Escherichia coli (E. coli*), it is problematic to dissolve the recombinant proteins which are embedded in inclusion bodies. In many cases, the proteins included in these inclusion bodies can efficiently be dissolved by using buffers containing 6 M guanidinium hydrochloride. This method is also particular useful for the isolation of affinity tagged proteins, e.g. when His-tag expression systems are used for the isolation and subsequent purification of recombinant affinity tagged proteins. The latter affinity purification procedure is based on the IMAC (Immobilized Metal Affinity Chromatography) technology, typically using a chelator such as NTA (nitrilotetraacetic acid), IDA (iminodiacetic acid) or TED (tris(carboxymethyl)ethylene diamine) with bound metal ions, for example nickel, copper, cobalt or zinc. Due to its successful application for protein purification IMAC became today's most widely used affinity chromatography technique (Block et al., Meth. Enzym. [2009] 463, 439-473). This method is often performed in the presence of 6 M guanidinium hydrochloride, resulting in the considerable advantages of high protein solubility, low unspecific binding and efficient protein purification. Proteins purified this way cannot directly be used for SDS polyacrylamide gel electrophoresis, e.g., for analysis of protein purity or expression level, due to the interference of the chaotropic salt guanidinium hydrochloride with the conditions used for SDS gel electrophoresis. This is mainly due to the formation of strong precipitates, generated upon addition of an SDS or LDS containing sample buffer and a running buffer for the gel analysis. Even low concentrations of guanidinium hydrochloride induce the formation of precipitates in the presence of SDS or LDS. The generally high ion concentration of the guanidinium hydrochloride containing buffers also impairs the running in and separation of the protein sample in established gel/buffer systems.

Conventional approaches therefore include diluting the sample containing GuHCl, prior to the addition of SDS-containing sample buffers to allow effective sample analysis. This procedure is rather ineffective as remaining amounts of even low concentrations of GuHCl still interfere with the SDS-PAGE. All constituents, e.g. proteins in the sample are also diluted with the sample, thus the detection of lower concentration of proteins is affected or disabled. The procedure is also time-consuming and laborious if it has to be carried out for a plurality of samples, e.g., when analyzing multiple fractions from a chromatography column. In another approach, very small amounts of sample per lane have been used, thereby minimizing the effect of the guanidine on the running properties of the gel. One way to do this was to dilute the GuHCl-containing sample before the addition of the SDS loading buffer. In this case the sample needs to be kept at a very low volume, e.g., as little as 1 microliter or less of the original sample, for gel loading. The resulting protein amount may be too low to allow detecting the protein of interest.

Alternatively, the denaturant has been changed to urea or SDS by dialysis, prior to loading the sample onto the gel. For example, dialysis against 8M urea buffer of SDS containing buffer, or exchange during a column washing step in affinity purification have been used. Complications of this approach include inter alia a potential lack of solubility of the protein in the alternative denaturants, loss of sample due to adhesion of protein to the materials used in this exchange methods (which unfortunately expose a very large surface area), and general quantitative as well as qualitative sample loss. This method is also time consuming and laborious, particularly if numerous samples have to be analyzed in parallel.

In another approach, urea rather than guanidine in the original denaturation step has been utilized. Then, the samples could be separated using SDS-PAGE, without the need to remove the urea. Urea is less efficient than guanidinium hydrochloride in solubilizing critical/hydrophobic/precipitated proteins, and considerably less effective in denaturing proteins. Solubilizing certain membrane proteins, in particular transmembrane proteins which span a plasma or organelle membrane, may require the use of detergents in addition to urea. Urea may also fail to solubilize very hydrophobic proteins in general. A solution of urea is known not to be stable. A urea solution will degrade spontaneously in a balance reaction, forming cyanate and ammonium. The cyanate ions generated are reactive with amino groups of proteins, neutralizing the positive charge of amines by carbamylation. This modification can affect stability, structure and function of a protein and is detrimental to further protein characterization, and to protein renaturation, and may preclude a subsequent use of proteins with its defined characteristics and activities. Cyanate ions may also impair protein purification, e.g. by IMAC.

Further, precipitation of proteins from solution of all samples i.e. by using trichloroacetic acid (TCA) precipitation or methanol/chloroform/water precipitation, followed by intensively washing and/or drying and subsequent addition to SDS-containing loading buffers can be carried out. These approaches are time intensive, especially when performed on a large number of samples. Loss of sample during precipitation and washing, especially of low molecular mass proteins typically occurs when employing these methods. Also, general loss of sample due to lack of re-dissolution of the precipitated protein, even in SDS-PAGE sample buffer under denaturing conditions, occurs in many cases and may lead to misinterpretation.

Finally, gel filtration or ion-exchange chromatography for which commercial kits are available can be used in order to remove a chaotropic agent such as guanidinium hydrochloride from a protein sample. Such an approach is time and cost intensive and involves the risk of precipitation of hydrophobic or non-soluble proteins during buffer exchange. Accordingly, such proteins will not be recovered from the chromatography resin. Another risk is sample-loss in general.

It would be advantageous to have available a method that allows sample, in particular protein sample, analysis where the sample contains a chaotropic agent, in particular guanidinium hydrochloride, guanidinium thiocyanate or other guanidinium salts.

### SUMMARY OF THE INVENTION

The present disclosure can be taken to generally relate to electrophoretic analysis, in particular gel electrophoretic analysis. A method described in this document allows direct electrophoretic analysis of matter such as proteins, which is included in a medium, for example an aqueous solution that contains a chaotropic agent, a guanidinium salt, and/or a potassium salt. A respective method is generally denaturing electrophoresis. A respective method allows such sample analysis without the need to remove the chaotropic agent, the guanidinium salt, and/or the potassium salt, by dialysis or chromatography or other methods described in the art, prior to analysis by denaturing electrophoresis. There is also no need for sample dilution or for using very small amounts of sample to keep the concentration of the chaotropic agent, guanidinium salt, and/or potassium salt in the protein sample at a low value.

A method as described herein can be applied to any gel electrophoresis and free flow electrophoresis system. In a method as described in this document N-Lauroylsarcosine is added to a sample that is to be electrophoretically analyzed. In embodiments where the tris/glycine electrophoresis system, also known as the Laemmli system, is applied, it is generally advantageous to use higher concentrations of buffer compounds than conventionally used.

In a first aspect, the invention provides a method of separating components of a sample by way of electrophoresis. The sample to be analyzed contains a chaotropic agent, a guanidinium salt, and/or a potassium salt. The method also includes adding N-Lauroylsarcosine to the sample. Furthermore the method may also include adding a reducing agent to the sample. A reducing agent is typically added in embodiments where denaturing electrophoresis is carried out (see below). The method also includes carrying out electrophoresis.

In typical embodiments of the method according to the first aspect electrophoresis is gel electrophoresis. In some embodiments of the method according to the first aspect electrophoresis is carried out using a cathode buffer that contains N-Lauroylsarcosine. In some embodiments of the method according to the first aspect the electrophoresis is denaturing electrophoresis, such as denaturing polyacrylamide gel electrophoresis (PAGE) or denaturing agarose gel electrophoresis. A respective gel electrophoresis is in some embodiments of the method according to the first aspect SDS-PAGE or SDS-agarose gel electrophoresis. Thus one or more electrophoresis buffer used include sodium dodecyl sulfate (SDS). In some embodiments an electrophoresis buffer used includes lithium dodecyl sulfate (LDS), and the electrophoresis is LDS-PAGE or LDS-agarose gel electrophoresis. In some embodiments where the electrophoresis is polyacrylamide gel electrophoresis and the electrophoresis gel is a polyacrylamide gel, the polyacrylamide gel includes an alkyl sulfate. In some embodiments where the electrophoresis is polyacrylamide gel electrophoresis, the running buffer and/or the sample buffer include(s) an alkyl sulfate. In some embodiments where the electrophoresis is agarose gel electrophoresis and the electrophoresis gel is an agarose gel, the agarose gel includes an alkyl sulfate. In some embodiments where the electrophoresis is agarose gel electrophoresis, the running buffer and/or the sample buffer include(s) an alkyl sulfate. The alkyl sulfate is included in an electrophoresis buffer. In some embodiments the respective electrophoresis buffer contains sodium decyl sulfate or sodium octyl sulfate.

The pH value of the sample to be analyzed may be of any value. In some embodiments the pH value of the sample is adjusted before exposing the sample to electrophoresis. In some embodiments the method includes adjusting the pH of the sample to a value of 6.5 or higher. In some embodiments the method includes adjusting the pH of the sample to a value of 7.5 or higher. In some embodiments the method includes adjusting the pH of the sample to a value of 8.0 or higher.

In some embodiments the chaotropic agent has ionic moieties and/or ionic properties. In some embodiments the chaotropic agent is a salt. In some embodiments the chaotropic agent, the guanidinium salt, and/or the potassium salt is different from a detergent. A detergent such as N-Lauroylsarcosine is an amphiphilic compound in that it includes polar and non-polar moieties. In contrast thereto, a chaotropic agent may be a polar molecule.

Typically the method according to the first aspect includes providing a sample, which contains the chaotropic agent, the guanidinium salt, and/or the potassium salt. The chaotropic agent may in some embodiments be a guanidinium salt. Since not all guanidinium salts are consistently regarded as chaotropic in the art, it is for sake of clarity separately defined that the method described herein can advantageously be used on any sample that contains a guanidinium salt. The same applies mutatis mutandis to a potassium salt. A chaotropic agent may for instance be one of guanidinium hydrochloride, guanidinium thiocyanate, sodium thiocyanate and potassium thiocyanate. In some embodiments the sample contains up to about 8 M of a chaotropic agent, a guanidinium salt and/or a potassium salt. The sample may for instance contain guanidinium hydrochloride at a concentration of up to 8 M. The chaotropic agent may in some embodiments be a potassium salt.

The sample is typically suspected to include one or more proteins and/or peptides. In some embodiments the sample is known to include one or more proteins and/or peptides.

In some embodiments the method according to the first aspect includes adding to the sample, which may be a protein sample and which includes the chaotropic agent, guanidinium salt, and/or potassium salt, an electrophoresis sample buffer. The electrophoresis sample buffer includes a buffer compound. The sample buffer further includes about 0.2 % (w/v) or more of N-Lauroylsarcosine. The electrophoresis sample buffer may for instance include about 1 % (w/v) or more of N-Lauroylsarcosine. In some embodiments the electrophoresis sample buffer also includes a metal chelating agent. The electrophoresis sample buffer furthermore typically includes a reducing agent, such as dithiothreitol or 2-mercaptoethanol (see also below). The reducing agent may be present at a concentration of about 10 mM or more, such as about 20 mM or more. In some embodiments a reducing agent is included in the electrophoresis sample buffer at a concentration of about 100 mM.

In some embodiments the electrophoresis is gel electrophoresis. In such an embodiment the sample, which includes the electrophoresis sample buffer, is furthermore being loaded to an electrophoresis gel. The electrophoresis gel may in some embodiments be a polyacrylamide gel. In some embodiments the electrophoresis gel may be a polyacrylamide gel. A method of these embodiments of the first aspect further includes exposing the electrophoresis gel to an electric field between an anode and a cathode. The anode is encompassed in an anode buffer. The cathode is encompassed in a cathode buffer. The cathode buffer includes about 0.003% (w/v) or more of N-Lauroylsarcosine. In some embodiments the method includes running the polyacrylamide gel in an electrophoresis running buffer that includes at least about 0.01% (w/v) N-Lauroylsarcosine.

In some embodiments the electrophoresis is free-flow electrophoresis, in which a liquid-based separation is performed, rather than a gel based separation as in gel electrophoresis. In such an embodiment a sample that includes N-Lauroylsarcosine is being introduced into a separation buffer. In some embodiments a sample which includes an electrophoresis sample buffer containing N-Lauroylsarcosine is being introduced into the separation buffer. In some embodiments the method includes electrophoresis in an electrophoresis separation buffer that includes about 0.003% (w/v) or more N-Lauroylsarcosine. In some embodiments the method includes performing electrophoresis in an electrophoresis separation buffer that includes about 0.01% to about 0.035 (w/v) or more N-Lauroylsarcosine. In some embodiments the method includes electrophoresis in an electrophoresis separation buffer that includes at least about 0.1 % to about 1% (w/v) or more N-Lauroylsarcosine. In some embodiments the method includes performing electrophoresis in an electrophoresis separation buffer that is of essentially the same composition as an electrophoresis running buffer described herein, containing about 0.003% (w/v) or more of N-Lauroylsarcosine. In some embodiments the method includes an electrophoresis separation buffer that is essentially identical in composition to the electrophoresis sample buffer described herein, containing N-Lauroylsarcosine. In some embodiments the method includes an electrophoresis separation buffer that is entirely identical in composition to the electrophoresis sample buffer described herein, containing N-Lauroylsarcosine. The sample may for example be injected into a thin buffer film. Insertion of the sample may be carried out over a certain period of time, such as several minutes or an hour, or continuously.

In some embodiments the concentration of N-Lauroylsarcosine in the sample buffer is about 2 % (w/v) or more. In some embodiments the sample buffer contains about 3 % (w/v) N-Lauroylsarcosine or more. In some embodiments the sample buffer contains about 3.5% (w/v) N-Lauroylsarcosine or more, such as about 4% (w/v) N-Lauroylsarcosine or more. Unless otherwise stated, all concentrations of components of the sample buffer refer to the final form of the sample buffer, in which it includes the sample to be analyzed.

The method of such embodiments may also include bringing the sample, to which the electrophoresis sample buffer has been added, to an elevated temperature for a preselected period of time, such as about 1 to about 10 minutes or longer. As an example, the sample may be exposed to an elevated temperature for about 5 minutes or more. The elevated temperature may be within the range from about 35°C to about 100°C, such as from about 40°C to about 100°C. In some embodiments the sample is brought, e.g. heated, to a temperature from about 65°C to about 75°C for about 5 to 10 minutes.

The metal chelating agent that may be included in the sample buffer may be present at a concentration of about 0.2 mM or more. In one embodiment the metal chelating agent is present at a concentration of about 3.0 mM. The sample buffer, which is added to the sample, may contain a buffer compound (cf. also below). The sample buffer contains N-Lauroylsarcosine. The sample buffer may further include one or more of a reducing agent and a metal chelating agent. It can further contain a loading aid and a tracking dye, as defined below. In some embodiments the sample buffer contains about 0.5 % (w/v) or more of N-Lauroylsarcosine. In some embodiments the concentration of N-Lauroylsarcosine in the sample buffer is about 3 % (w/v) or more. In some embodiments the sample buffer contains a metal chelating agent. Such a metal chelating agent, herein also referred to as a chelating agent, may be at a concentration of about 3.0 mM or more. In some embodiments the sample buffer contains a reducing agent. A respective reducing agent may be present in the sample buffer at a concentration of about 10 mM or more, such as about 20 mM or more. In some embodiments the reducing agent is present at a concentration of about 50 mM or more.

In some embodiments of the method according to the first aspect the reducing agent, which is being added, includes at least one of 2-mercaptoethanol, dithiothreitol (DTT), tris(2-carboxyethyl)phosphine (TCEP), dithioerythritol (DTE), reduced glutathione, cysteamine, tri-n-butylphosphine (TBP), dithioerythriol, 2-mercaptoethylamin for instance in the form of 2-mercaptoethylamin-HCl, dithiobutylamine (DTBA), cysteine, cysteine-thioglycolate, thioglycolic acid and hydroxyethyldisulphide (HED). In one embodiment the reducing agent is or includes dithioerythritol (DTE). In one embodiment the reducing agent is or includes (2S)-2-amino-1,4-dimercaptobutane, abbreviated dithiobutylamine (DTBA). The reducing agent is or includes in one embodiment 1,4-dithio-D-threitol (DTT), also known as Cleland's reagent. As an illustrative example, DTT may be added to the sample, for instance via a sample buffer, and the concentration of DTT in the sample buffer may be about 10 mM or higher. As already noted above, except when stated otherwise, all concentrations of components of the sample buffer, including the reducing agent, refer to the final form of the sample buffer, in which the sample to be analyzed is included in the sample buffer.

In one embodiment the sample buffer has a pH value of about 8.45. The buffer compound included in the sample buffer is or includes in some embodiments tris (tris(hydroxymethyl)aminomethane, or 2-amino-2-hydroxymethyl-propane-1,3-diol). In some embodiments the metal chelating agent is or includes EDTA. In one embodiment the metal chelating agent is EDTA and the reducing agent is DTT.

Any electrophoresis running buffer may be used for carrying out electrophoresis as disclosed herein as long as the cathode running buffer contains N-Lauroylsarcosine. The electrophoresis running buffer used for performing electrophoretic separation may in some embodiments include two electrophoresis buffers, one for the cathode and one for the anode. These two buffers may be identical or different. The electrophoresis running buffer used for performing electrophoretic separation has in some embodiments a pH value of about 6.7 or more. In some embodiments the pH value of the running buffer is about 7.2 or more. In some embodiments the pH value of the running buffer is about 7.7 or more. The running buffer may in some embodiments contain N-Lauroylsarcosine. In some embodiments, the running buffer may contain N-Laurylsarcosine in an amount of about 0.003 % (w/v) to about 0.05% (w/v) or more. In some embodiments the running buffer contains about 0.05 M tris. The running buffer contains in some embodiments about 0.05 M 3-(N-morpholino)propanesulfonic acid (MOPS). In some embodiments the running buffer contains about 0.1% (w/v) SDS. The running buffer may also contain about 0.5 mM to about 1.5 mM EDTA. In some embodiments the running buffer may contain about 1.0 mM EDTA or more. In some embodiments the cathode running buffer contains about 1mM sodium bisulfite. Such a cathode running buffer is typically employed for carrying out electrophoresis of gel systems buffered with Bis(2-hydroxyethyl)amino-tris(hydroxymethyl)methane (Bistris). In some embodiments both the cathode running buffer and the anode running buffer contain about 1mM sodium bisulfite.

In some embodiments the electrophoresis running buffer used for performing electrophoretic separation has a pH value of about 7.0 or more. As noted above, the running buffer, at least the cathode running buffer, contains N-Laurylsarcosine. The running buffer may contain N-Laurylsarcosine in an amount from about 0.003 % (w/v) to about 0.06 % (w/v). In some embodiments the cathode running buffer may contain N-Lauroylsarcosine in an amount from 0.01 % to about 0.033 % (w/v). In some embodiments the running buffer contains about 0.025 M tris. The running buffer contains in some embodiments about 192 mM glycine. In some embodiments the running buffer contains about 0.1% (w/v) a detergent such as SDS or LDS. The running buffer may also contain about 0.5 mM to 1.5 mM EDTA or more. Such a running buffer is typically employed for electrophoresis according to the tris/glycine gel electrophoresis system. In such embodiments gel electrophoresis may be carried out, employing a gel buffer that has a pH value of about 7.8 or more. A respective gel buffer may contain tris in a final concentration of about 0.4 M or more. In some embodiments such a gel buffer contains about 0.7 M tris or more. In some embodiments a respective gel buffer may contain about 1.125 M tris or more, as final concentration. In some embodiments the gel buffer contains about 0.1% (w/v) SDS. In some embodiments this gel buffer may be used for both the stacking gel and the separating gel.

As noted above, in some embodiments two different running buffers are used, one for the cathode and one for the anode. Such a system may for instance be applied for carrying out electrophoresis according to the Schaegger system. The anode running buffer may have a pH value of about 10.0 or less. In some embodiments the anode running buffer may have a pH value of about 7.2 or more. The anode running buffer may contain about 100 mM tris. The cathode running buffer may have a pH value of about 7.2 or more. The cathode running buffer may in some embodiments have a pH value of about 10.0 or less. The cathode running buffer may contain about 100 mM tris. Furthermore the cathode running buffer may contain about 100 mM tricine. The cathode running buffer may also contain EDTA, for instance in a concentration of about 0.5 to about 1.5 mM. In some embodiments about 1.0 mM or more of EDTA is included in the cathode running buffer. In some embodiments the anode running buffer may contain about 1.5 mM EDTA. The anode running buffer may also contain N-Lauroylsarcosine in an amount from 0.01 % to about 0.033 % (w/v).

In some embodiments, the cathode running buffer contains about 0.003 % (w/v) or more of N-Lauroylsarcosine. In some embodiments the cathode running buffer may contain N-Lauroylsarcosine in an amount of about 0.008 % (w/v) or more. In some embodiments the cathode running buffer may contain N-Lauroylsarcosine in an amount from about 0.01 % to about 0.033 % (w/v). Such a combination of running buffers is typically employed for electrophoresis according to the tris/tricine system. In such embodiments a gel buffer may be used that has a pH value of about 8.4 or more. Such a gel buffer may include about 1.0 M tris. The gel buffer may also contain about 0.1% (w/v) of a detergent such as SDS or LDS. In some embodiments this gel buffer may be used for both the stacking gel and the separating gel. Unless otherwise stated, all concentrations of components of the running buffers and gel buffers refer to the final form of these buffers, in which they include all constitutents of the respective gels and running buffers.

In a second aspect, the invention provides an electrophoresis sample buffer. The electrophoresis sample buffer contains N-Lauroylsarcosine. The sample buffer also contains a buffer compound. In some embodiments the electrophoresis sample buffer also contains a reducing agent.

In some embodiments the electrophoresis sample buffer according to the second aspect has a pH value of about 8.0 or higher.

In some embodiments of the electrophoresis sample buffer according to the second aspect the reducing agent includes at least one of 2-mercaptoethanol, dithiothreitol (DTT), tris(2-carboxyethyl)phosphine (TCEP), dithioerythritol (DTE), reduced glutathione, cysteamine, tri-n-butylphosphine (TBP), dithioerythriol, 2-mercaptoethylamin for instance as 2-mercaptoethylamin-HCl, dithiobutylamine (DTBA), cysteine, cysteine-thioglycolate, thioglycolic acid, and hydroxyethyldisulphide (HED).

In some embodiments the electrophoresis sample buffer according to the second aspect further includes a metal chelating agent. The metal chelating agent may in some embodiments be or include one or more of ethylenediaminotetraacetic acid (EDTA), ethylene glycol tetraacetic acid (EGTA), diaminocyclohexanetetraacetic acid (DCTA), nitrilotriacetic acid (NTA), diethylenetriamine pentaacetate (DTPA), diethyldithiocarbamate (DEDTC), 1,2-bis(o-aminophenoxy)-ethane-N,N,N',N'-tetraacetic acid (BAPTA), glutamic acid diacetic acid (GLDA), and tripolyphosphate (TPP).

In one embodiment the electrophoresis sample buffer according to the second aspect includes about 0.2% (w/v) or more of N-Laurylsarcosine, a metal chelating agent at a concentration of about 0.5 mM or more, and a reducing agent at a concentration of about 10 mM or more. In some embodiments the sample buffer includes about 2% (w/v) or more of N-Laurylsarcosine. In some embodiments the sample buffer includes a metal chelating agent. The metal chelating agent is in some embodiments included in the sample buffer at a concentration of 0.6 mM or more, such as about 1 mM or more. The metal chelating agent may for instance be present at a concentration of about 3.0 mM or more. In some embodiments the sample buffer includes a reducing agent at a concentration of about 100 mM or more. In one embodiment the electrophoresis sample buffer contains about 0.5 % (w/v) or more of N-Lauroylsarcosine, a metal chelating agent at a concentration of about 3.0 mM, and a reducing agent at a concentration of about 100 mM.

In some embodiments the buffer compound included in the electrophoresis sample buffer is tris. In one embodiment the electrophoresis sample buffer has a pH of about 8.5 and contains about 0.063 M tris, about 3% (w/v) N-Lauroylsarcosine, about 20% (v/v) Glycerol, about 100 mM DTT, about 0.001% (w/v) BPB and about 3 mM EDTA. As noted above, except when stated otherwise, all concentrations of components of the sample buffer, including the reducing agent, refer to the final form of the sample buffer, in which the sample to be analyzed is included in the sample buffer.

In some embodiments the buffer compound contained in the electrophoresis sample buffer of the second aspect is at a concentration of about 230 mM or less. In some embodiments the buffer compound is included in the sample buffer at a concentration of about 200 mM or less. In some embodiments the buffer compound is included in the sample buffer at a concentration of about 150 mM or less. The sample buffer contains in some embodiments more than about 2% (w/v) N-Lauroylsarcosine.

In some embodiments the electrophoresis sample buffer according to the second aspect is provided as a stock solution. In these embodiments the above buffer components are included in the electrophoresis sample buffer in several-fold higher concentrations, albeit the relative amounts of the buffer components may be essentially the same as indicated above. In some embodiments a 10x stock solution may be provided. In such an embodiment the electrophoresis sample buffer may include about 2% (w/v) or more of N-Laurylsarcosine, and a buffer compound such as tris, triethanolamine, tricine, Bistris, or imidazole. The buffer compound may for instance be included in the stock solution at a concentration of about 0.5 M. A respective 10x stock solution may further include a metal chelating agent at a concentration of about 5 mM or more. In some embodiments such a 10x stock solution may also include a reducing agent at a concentration of about 100 mM or more. The sample buffer may for example include about 20% (w/v) or more of N-Laurylsarcosine. The sample buffer may also include a metal chelating agent at a concentration of about 30 mM or more. As a further example, in some embodiments a 5x stock solution may be provided. The electrophoresis sample buffer may in such an embodiment include about 1% (w/v) or more of N-Laurylsarcosine, and a buffer compound, for instance at a concentration of about 0.25 M. Such a 5x stock solution may further include a metal chelating agent at a concentration of about 2.5 mM or more, and/or a reducing agent at a concentration of about 50 mM or more. The sample buffer may for instance include about 10% (w/v) or more of N-Laurylsarcosine. In some embodiments the sample buffer includes a metal chelating agent at a concentration of about 15 mM or more. In some embodiments the sample buffer in form of a stock solution includes a reducing agent at a concentration of about 500 mM or more.

In a third aspect the invention provides a gel electrophoresis gel buffer for analysis of a sample in a tris/glycine system, where the sample contains a chaotropic agent. This buffer is accordingly a gel buffer for gel electrophoretic analysis, using the tris/glycine system, of a respective sample. The gel electrophoresis gel buffer of the third aspect contains a buffer compound. The buffer compound typically includes tris, which may in some embodiments be present in an amount of about 0.3 M or more. In some embodiments the buffer compound is present in an amount of about 1 M or more.

The gel electrophoresis gel buffer of the third aspect in some embodiments furthermore contains a detergent. The detergent may for instance be SDS, which may be present in an amount of about 0.1 % (w/v) or more.

In some embodiments the gel electrophoresis gel buffer according to the third aspect has a pH value of about 7.5 or more. In some embodiments the gel electrophoresis gel buffer according to the third aspect has a pH value of about 8.0 or more. In some embodiments the gel electrophoresis gel buffer according to the third aspect has a pH value of about 8.8 or more. In some embodiments the gel buffer contains about 1.125 M tris or more. In some embodiments the gel buffer contains about 1 M tris or more, about 0.1% (w/v) SDS or more, and has a pH value of about 8.4 or more. A gel buffer of such embodiments may be used for electrophoresis according to the tris/glycine system or according to the tris/tricine system. The gel buffer may be used for both the stacking gel and the separating gel. In some embodiments the gel buffer contains about 1.1 M tris or more, about 0.1% (w/v) SDS or more, and has a pH value of about 8.6 or more.

In some embodiments the gel electrophoresis gel buffer according to the third aspect is provided as a stock solution. In these embodiments the above buffer components are included in the gel buffer in several-fold higher concentrations, albeit the relative amounts of the buffer components may be essentially the same as indicated above. In some embodiments a 5x stock solution may be provided. In such an embodiment the gel buffer may include about 0.5 % (w/v) or more of a detergent, and a buffer compound in an amount of about 1.5 M or more. In some embodiments a respective 5x stock solution may contain about 2.5 M or more of a buffer compound. In some embodiments a 10x stock solution may be provided. In such an embodiment the gel buffer may include about 1 % (w/v) or more of a detergent, and a buffer compound in an amount of about 3 M or more. The gel buffer provided as a stock solution may contain about 10 M tris or more, about 1% (w/v) SDS or more, and have a pH value of about 8.4 or more.

In a fourth aspect, the invention relates to the use of an electrophoresis sample buffer according to the second aspect for separation of components of a sample by electrophoresis, including gel electrophoresis. In some embodiments the use is for separation of components of a sample by denaturing gel electrophoresis. In some embodiments the sample to be analyzed by electrophoresis, such as denaturing gel electrophoresis, includes a chaotropic agent.

In a fifth aspect the invention provides a gel electrophoresis running buffer. The gel electrophoresis running buffer includes a buffer compound. The electrophoresis running buffer has a pH value of about 7.5 or more. In some embodiments the electrophoresis running buffer has a pH value of about 8.0 or more. A pH value of about 8.0 or more may for example be used when electrophoresis is performed according to the tris/glycine system. The gel electrophoresis running buffer may also include about 0.003% (w/v) or more N-Lauroylsarcosine, including e.g. about 0.01 % (w/v) or more N-Lauroylsarcosine. In some embodiments the gel electrophoresis running buffer furthermore includes about 1 mM or more of a metal chelating compound such as EDTA. In some embodiments the gel electrophoresis running buffer furthermore includes about 0.05 % or more of a detergent such as SDS. In some embodiments the gel electrophoresis running buffer furthermore includes about 0.1 % or more of a detergent. In some embodiments the running buffer contains about 0.1% (w/v) SDS. In some embodiments a buffer compound included in the electrophoresis running buffer is tris. The buffer compound may be included in the gel electrophoresis running buffer in an amount of about 0.02 M or more. In some embodiments the running buffer contains about 0.025 M tris. The running buffer contains in some embodiments about 0.192 M glycine.

The gel electrophoresis running buffer according to the fifth aspect has in some embodiments a pH value of about pH 7.0 or more. In some embodiments the running buffer has a pH value of about 7.7 or more. In some embodiments the running buffer contains about 0.05 M tris. The running buffer contains in some embodiments about 0.05 M MOPS. The running buffer may also contain about 1 mM EDTA or more. In some embodiments the running buffer contains about 1 mM or more of sodium bisulfite. In one embodiment the running buffer contains about 0.05 M tris or more, about 0.05 M MOPS, about 1 mM EDTA or more, about 0.01 % (w/v) or more N-Lauroylsarcosine, about 1 mM sodium bisulfite, and has a pH value of about 7.7 or more. Such a running buffer is typically employed for electrophoresis according to the Bistris gel system.

In some embodiments the gel electrophoresis running buffer according to the fifth aspect has a pH value of about 8.3 or more. In some embodiments the running buffer contains about 0.025 M tris or more. The running buffer may contain about 190 mM glycine. In one embodiment the running buffer contains about 0.025 M tris or more, about 192 mM glycine, about 0.1 % (w/v) SDS, about 1.5 mM EDTA, about 0.01 % (w/v) or more N-Lauroylsarcosine, and has a pH value of about 8.3 or more. Such a running buffer is typically employed for electrophoresis according to the tris/glycine system.

In some embodiments the gel electrophoresis running buffer according to the fifth aspect includes two buffers, one for the cathode and one for the anode. These two buffers are in some embodiments different. The cathode buffer has in some embodiments a pH value of about 8.0 or more. The cathode running buffer may contain about 100 mM tris. Furthermore the cathode running buffer may contain about 100 mM tricine. The cathode running buffer may also contain about 1.5 mM EDTA. In some embodiments the cathode running buffer may contain N-Lauroylsarcosine in an amount of about 0.01 % to about 0.033 % (w/v). In some embodiments the cathode running buffer contains N-Lauroylsarcosine in an amount of about 0.003 % (w/v) or more. In one embodiment the cathode buffer contains about 0.1 M tris or more, about 0.1M tricine, about 1.5 mM EDTA, about 0.1 % (w/v) SDS, about 0.01 % (w/v) or more N-Lauroylsarcosine, and has a pH value of about 8.2 or more. Such a cathode buffer is typically employed for electrophoresis according to the tris/tricine system. The anode buffer has in some embodiments a pH value of about 8.8 or more. The anode running buffer may contain about 100 mM tris. Such an anode buffer is typically employed for electrophoresis according to the tris/tricine system.

In some embodiments the gel electrophoresis running buffer according to the fifth aspect is provided as a stock solution. In these embodiments the above buffer components are included in the running buffer in several-fold higher concentrations, albeit the relative amounts of the buffer components may be essentially the same as indicated above. In some embodiments a 5x stock solution may be provided. In such an embodiment the gel buffer may include about 0.05 % (w/v) or more N-Lauroylsarcosine. The buffer compound may be included in such a gel electrophoresis running buffer in an amount of about 0.1 M or more. In some embodiments the gel electrophoresis running buffer in the form of a 5x stock solution includes about 5 mM or more of a chelating compound such as EDTA. A respective gel electrophoresis running buffer furthermore includes in some embodiments about 0.25 % or more of a detergent.

In a sixth aspect the invention provides an electrophoresis separation buffer. Such an electrophoresis separation buffer is typically used in free-flow electrophoresis. The electrophoresis separation buffer includes a buffer compound. The electrophoresis separation buffer has a pH value of about 6.5 or more. The electrophoresis may in some embodiments have a pH value of about 7.2 or more. In some embodiments the electrophoresis separation buffer has a pH value of about 8.0 or more. The electrophoresis separation buffer may also include about 0.003% (w/v) or more N-Lauroylsarcosine, including e.g. about 0.01 % (w/v) or more N-Lauroylsarcosine. In some embodiments the electrophoresis separation buffer includes about 0.01% to about 0.035 (w/v) or more of N-Lauroylsarcosine. In some embodiments the electrophoresis separation buffer includes about 0.2% (w/v) or more, such as to about 1% (w/v) or more of N-Lauroylsarcosine. In some embodiments the electrophoresis separation buffer includes about 3% (w/v) or more of N-Lauroylsarcosine. In some embodiments the electrophoresis separation buffer furthermore includes about 1 mM or more of a chelating compound such as EDTA. In some embodiments the electrophoresis separation buffer includes about 10 mM or more more, including 30mM or more of a reducing agent. In some embodiments the electrophoresis separation buffer furthermore includes about 50 mM to 100 mM or more of DTT. In some embodiments the electrophoresis separation buffer furthermore includes about 0.05 % or more of a detergent such as SDS. In some embodiments the electrophoresis separation buffer furthermore includes about 0.1 % or more of a detergent. In some embodiments the separation buffer contains about 0.1% (w/v) SDS. In some embodiments a buffer compound included in the electrophoresis separation buffer is tris. The buffer compound may be included in the electrophoresis separation buffer in an amount of about 0.02 M or more. In some embodiments the separation buffer contains about 0.025 M tris. The separation buffer contains in some embodiments about 0.192 M glycine. The electrophoresis separation buffer contains in some embodiments about 0.05 M tris. The separation buffer contains in some embodiments about 0.05 M MOPS. The electrophoresis separation buffer contains in some embodiments about 0.1 M tris. The separation buffer contains in some embodiments about 0.1 M Tricine. In some embodiments the electrophoresis separation buffer may also contain glycerol in a concentration of about 5% to about 25% or more.

In a seventh aspect, the invention relates to the use of an electrophoresis sample buffer according to the second aspect for separation of components of a sample by gel electrophoresis, such as denaturing gel electrophoresis. In some embodiments the sample to be analyzed by gel electrophoresis includes a chaotropic agent, a guanidinium salt, and/or a potassium salt. In some embodiments the electrophoresis sample buffer is used for the tris/glycine gel electrophoresis system. The electrophoresis sample buffer is in some embodiments used for the Bistris based gel electrophoresis system. In some embodiments the electrophoresis sample buffer is used for a gel electrophoresis system that differs from the known Bistris gel electrophoresis system as used in the art. In some embodiments the electrophoresis sample buffer is used for a gel electrophoresis system that is not a Bistris based gel electrophoresis system. In some embodiments the electrophoresis sample buffer is used for a tris/tricine based gel electrophoresis system

In an eighth aspect the invention provides a kit for the separation of components of a sample by gel electrophoresis, such as denaturing gel electrophoresis. The sample to be analyzed by gel electrophoresis typically includes a chaotropic agent. The kit includes a gel electrophoresis sample buffer according to the second aspect, a stock solution for preparing a gel electrophoresis sample buffer according to the second aspect, or the components of the gel electrophoresis sample buffer in dry form. The components of the gel electrophoresis sample buffer in dry form are defined by a mixture of N-Lauroylsarcosine, a reducing agent, and a buffer compound. The kit may also include a running buffer according to the fifth aspect. The running buffer includes N-Lauroylsarcosine. The kit may also include a gel buffer for the tris/glycine system according to the third aspect.

In a ninth aspect the invention relates to the use of a kit according to the eighth aspect for separation of components of a sample by gel electrophoresis. In some embodiments the gel electrophoresis is denaturing gel electrophoresis. In some embodiments the sample to be analyzed by gel electrophoresis includes a chaotropic agent, a guanidinium salt, and/or a potassium salt.

In a tenth aspect the invention relates to the use of an electrophoresis running buffer for the electrophoretic analysis of a sample. The sample contains a guanidinium salt, a potassium salt and/or a chaotropic agent. The gel electrophoresis running buffer has a pH value of about 7.0 or more. In some embodiments the gel electrophoresis running buffer has a pH value of about 7.5 or more. The gel electrophoresis running buffer contains a buffer compound as well as about 0.01 % (w/v) or more of N-Lauroylsarcosine. In some embodiments the gel electrophoresis running buffer contains about 0.03 % (w/v) or more of N-Lauroylsarcosine. The buffer compound included in the gel electrophoresis running buffer may for example be tris. In some embodiments the gel electrophoresis running buffer contains about 0.1 % (w/v) or more of a detergent. In some embodiments the gel electrophoresis running buffer is a gel electrophoresis running buffer according to the fifth aspect.

The summary of the invention described above is non-limiting and other features and advantages of the invention will be apparent from the following detailed description of the invention, and from the claims.

### BRIEF DESCRIPTION OF THE DRAWINGS

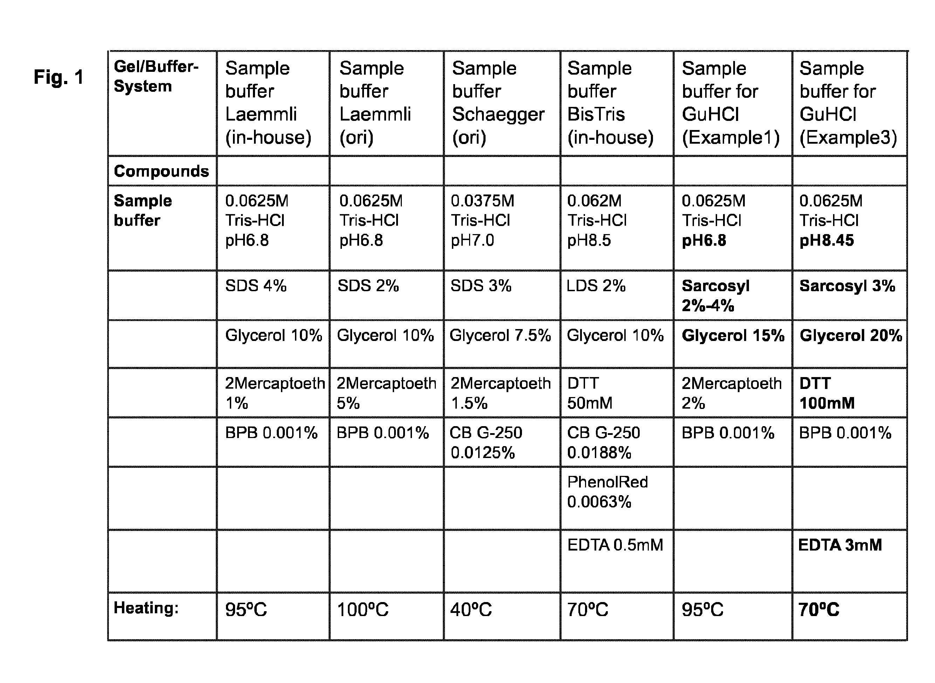

Figure 1 depicts examples of suitable compositions of a sample buffer for different electrophoresis systems. Examples of sample buffer conditions are shown in comparison to several SDS-sample buffers of gel electrophoresis systems known in the art. Illustrated are typical final concentrations after an addition of a sample, as well as typical heating temperatures as used for protein denaturation of a sample.

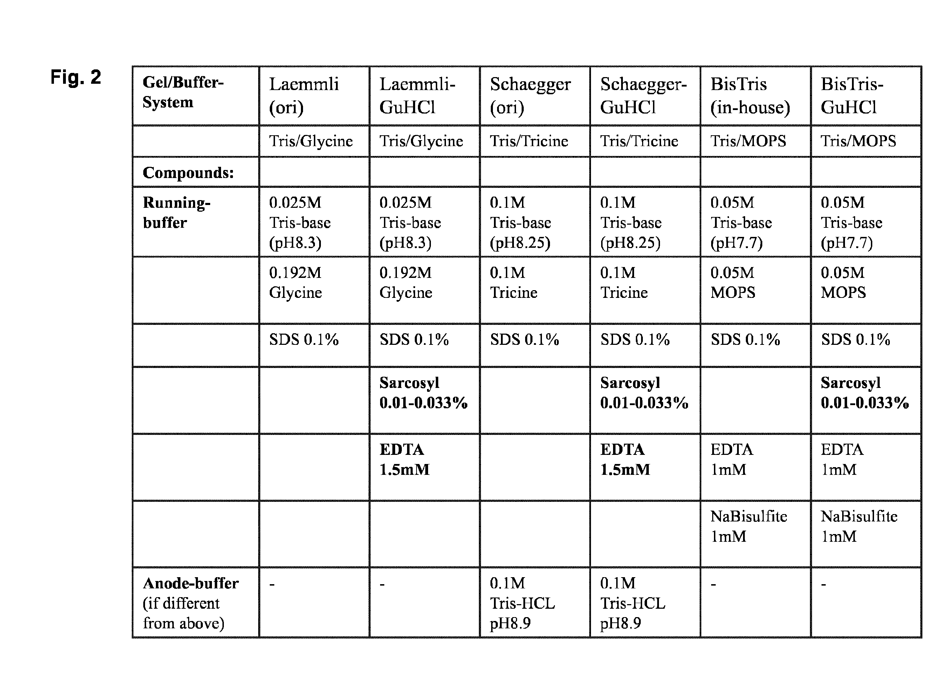

**Figure 2** depicts examples of suitable compositions of running buffers for different gel electrophoresis systems. An anode running buffer is defined separately where it differs in composition or concentration from the cathode running buffer. Illustrated are typical final concentrations as used during gel electrophoresis.

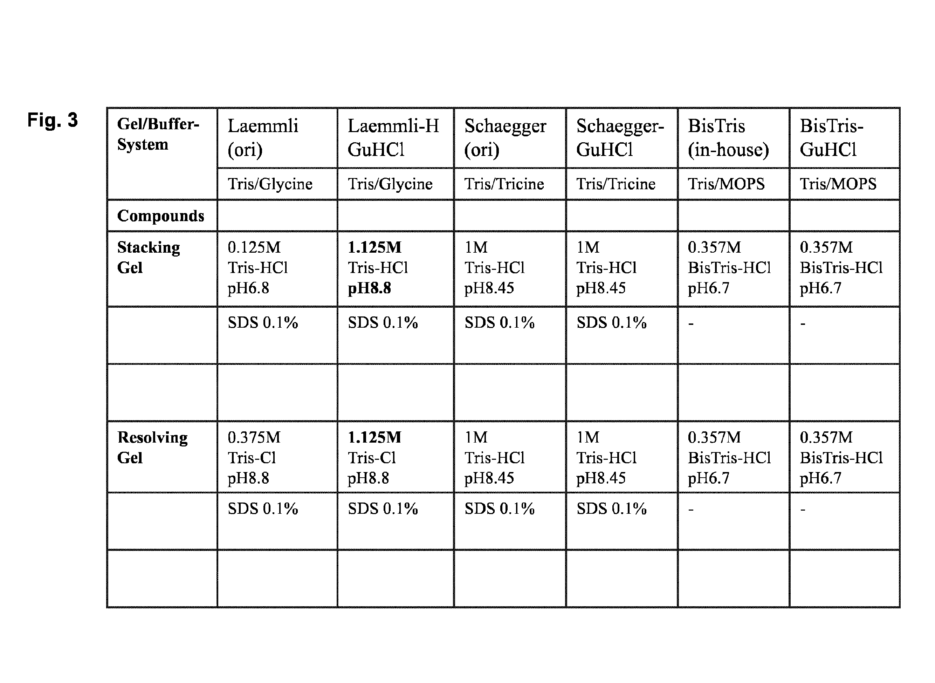

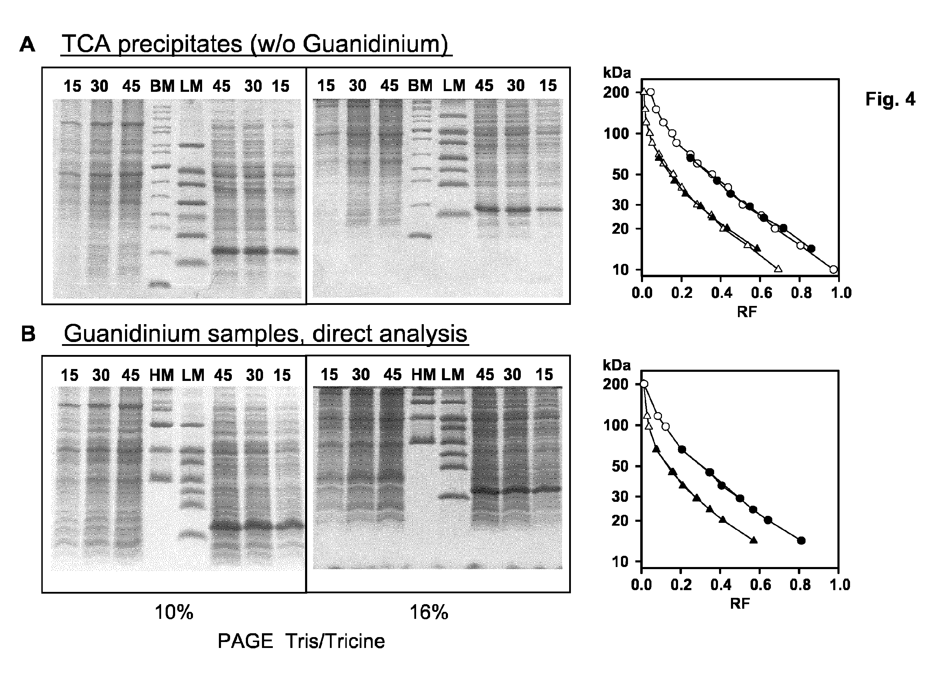

**Figure 3** depicts examples of suitable compositions of gel buffers for different gel electrophoresis systems. Illustrated are typical final concentrations in preparations of the electrophoresis gel. Such buffer conditions were applied to prepare exemplary polyacrylamide gels used in Figures 4 to 13.
**Figure 4** shows polyacrylamide gel electrophoretic separations using tris/tricine-PAGE (10% and 16%) under denaturing conditions. **B:** Samples from bacterial extracts containing 6 M guanidinium hydrochloride were directly used for electrophoresis under conditions as described herein. **A:** In an identical set of samples guanidinium hydrochloride was depleted by precipitation using the TCA method, dried and neutralized before electrophoresis under established buffer conditions commonly used in the art. A comparison of the relative mobility (RF) of standard proteins (BM, LM, HM) is shown in the calibration curves to the right. Open and filled circles: BM/HM and LM in 10% gels; open and filled triangles: BM/HM and LM in 16% gels.

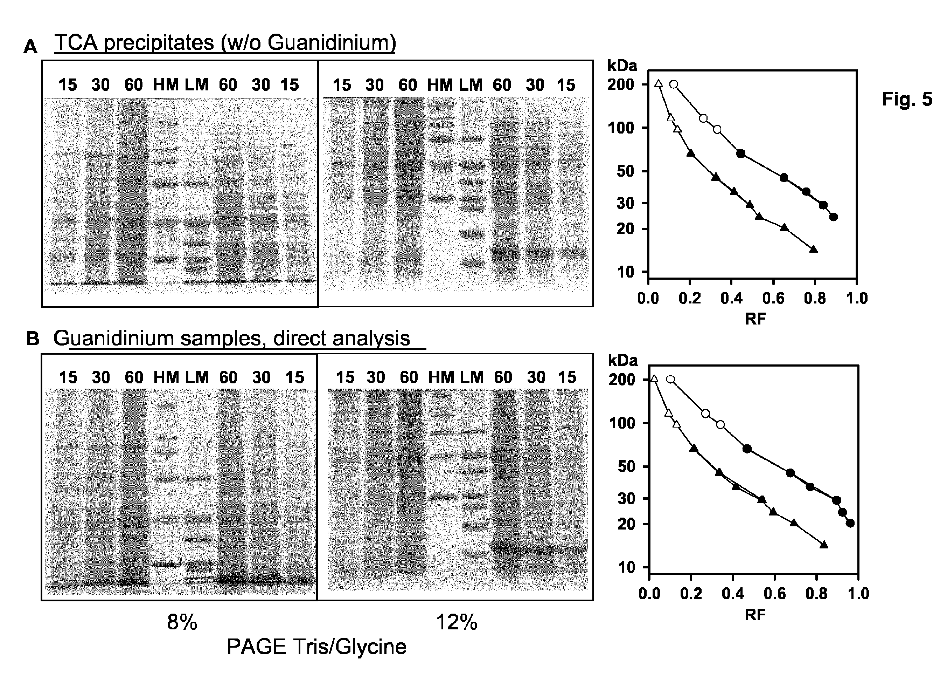

**Figure 5** shows polyacrylamide gel electrophoretic separations using tris/glycine-PAGE (8% and 12%) under denaturing conditions. **B:** Samples from bacterial extracts containing 6 M guanidinium hydrochloride were directly used for electrophoresis under conditions as described herein. **A:** In an identical set of samples guanidinium hydrochloride was depleted by precipitation using the TCA method, dried and neutralized before electrophoresis under established buffer conditions known in the art. A comparison of the relative mobility (RF) of standard proteins (HM, LM) is shown in the calibration curves to the right. Open and filled circles: HM and LM in 8% gels; open and filled triangles: HM and LM in 12% gels.

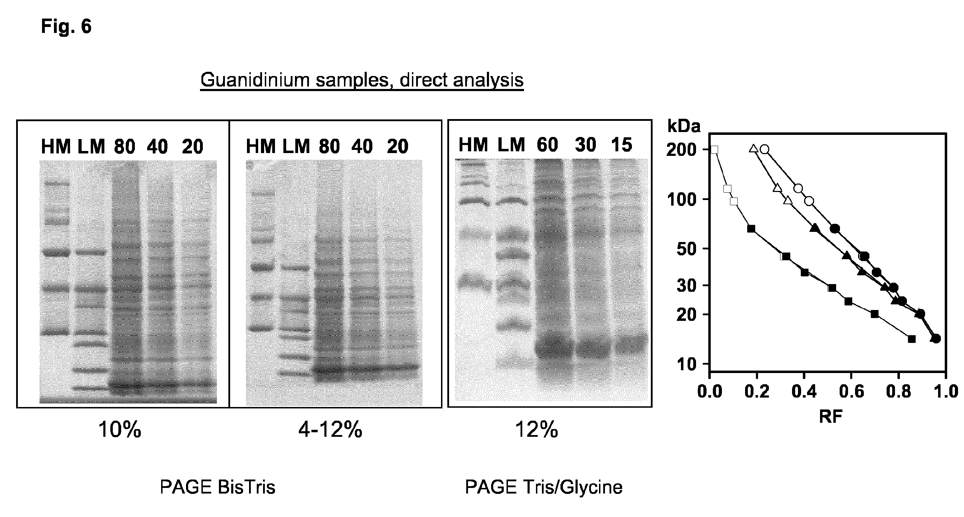

**Figure 6** depicts the separation of samples that contain guanidinium hydrochloride as the chaotropic agent in different systems: precast Criterion™ XT Bistris gel system (from Bio-Rad Laboratories GmbH, Munich, Germany) (10% and 4%-12% gradient polyacrylamide), as well as a standard tris-HCl buffered gel according to Laemmli without modification (12% polyacrylamide). Samples from bacterial extracts containing 6 M guanidinium hydrochloride were directly used for electrophoresis as described herein. The calibration curves to the right illustrate the relative mobility (RF) of standard proteins (HM, LM). Open and filled circles: HM and LM in 4-12 % BisTris gel; open and filled triangles: HM and LM in 10% Bistris gel; open and filled rectangles: HM and LM in 12% tris/glycine. Please note that the full performance of a tris/glycine-PAGE as disclosed herein is demonstrated in Figure 5.

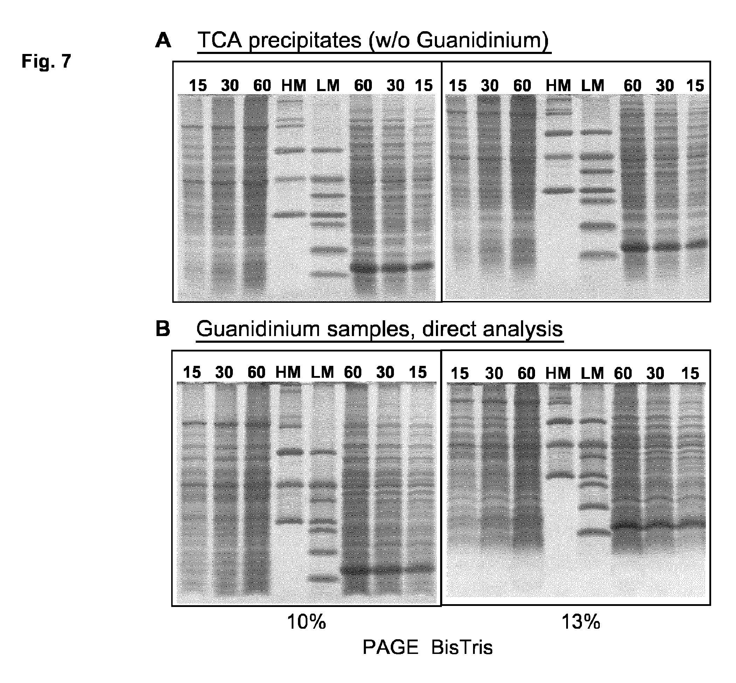

**Figure 7** depicts electrophoretic separations using the Bistris gel system (10% and 13%) with a tris/MOPS running buffer under denaturing conditions. **B:** Samples from bacterial extracts containing 6 M guanidinium hydrochloride were directly used for electrophoresis under conditions as described herein. **A:** In an identical set of samples guanidinium hydrochloride was depleted by precipitation using the TCA method, dried and neutralized before electrophoresis under established buffer conditions known in the art. HM and LM indicate loaded standard protein mixtures.

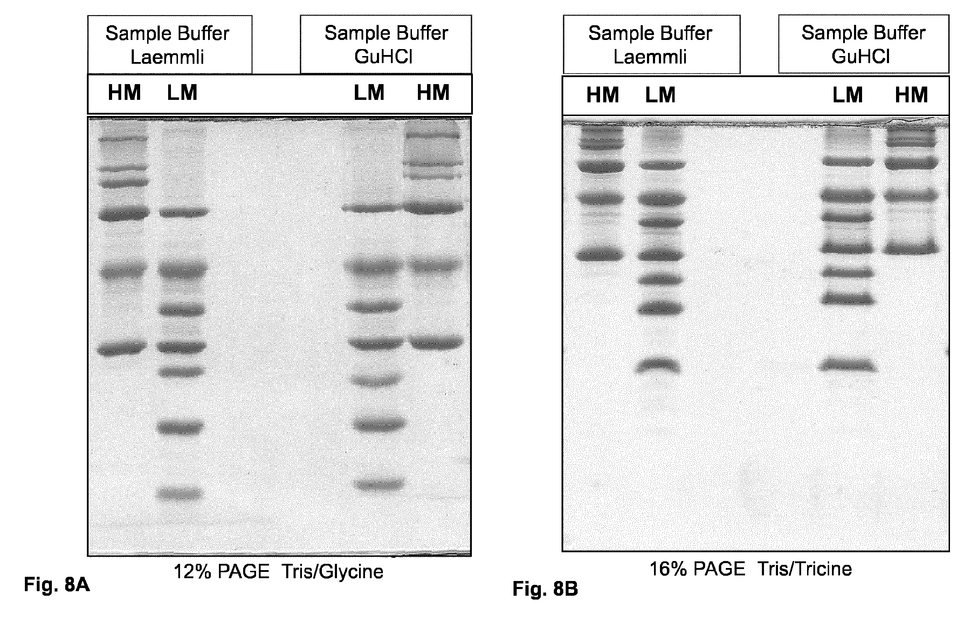

**Figure 8** illustrates the electrophoretic performance of a sample buffer as described herein, and a standard SDS-sample buffer according to Laemmli. Standard protein mixtures of high molecular mass (HM) and low molecular mass (LM), were loaded with the indicated sample buffer. PAGE was performed using the tris/glycine system (**A**, 12%, according to Laemmli) or the tris/tricine system (**B**, 16%, according to Schaegger).

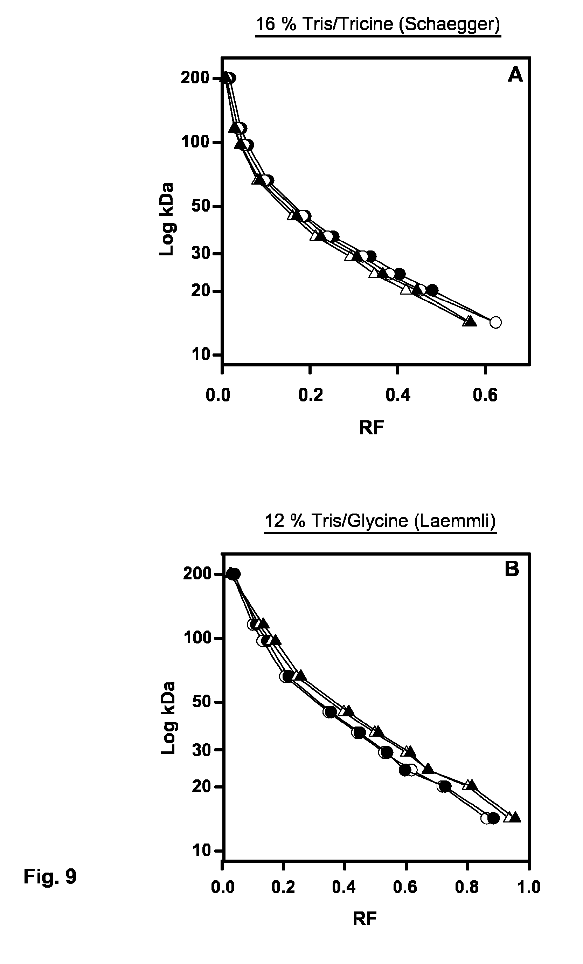

**Figure 9** illustrates the electrophoretic performance of a sample buffer and a running buffer with addition of N-Lauroylsarcosine as disclosed herein, compared to a standard SDS-sample buffer and SDS-running buffer according to Laemmli (**B**, 12% polyacrylamide gel) or Schaegger (**A**, 16% polyacrylamide gel), respectively. Open circles: sample buffer containing N-Lauroylsarcosine, running buffer without N-Lauroylsarcosine; Filled circles: sample buffer and running buffer according to Laemmli, without N-Lauroylsarcosine; Open triangles: sample buffer containing N-Lauroylsarcosine, running buffer with N-Lauroylsarcosine; Filled triangles: sample buffer according to Laemmli without N-Lauroylsarcosine, running buffer with N-Lauroylsarcosine. The calibration curves illustrate the relative mobilitiy (RF) of standard proteins (HM, LM), demonstrating almost no differences in the electrophoretic separation characteristics under the influence of N-Lauroylsarcosine according to the invention, compared to conditions according to established PAGE procedures.

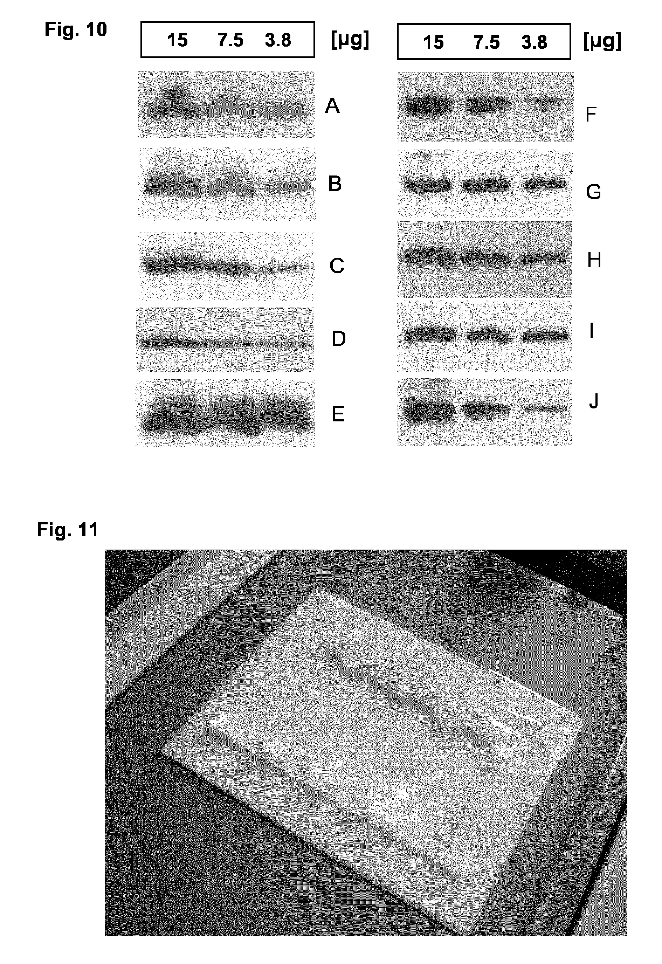

**Figure 10** depicts an immunochemical detection of specific recombinant 6xHis-tagged proteins from crude bacterial extracts in 6M guanidinium hydrochloride. Semi-Dry Western Blot was performed after samples were directly loaded and separated using the tris/glycine-PAGE, tris/tricine-PAGE or Bistris-PAGE under denaturing conditions as disclosed herein. The following proteins were detected after a transfer to nitrocellulose membranes by specific antibodies: A = Anti-S100A9, tris/tricine system, 16 %; B = Anti-S100A9, tris/tricine system, 10 %; C = Anti-S100A9, tris/glycine system, 12 %; D = Anti-S100A9, tris/glycine system, 8 %; E = Anti-S100A9, Bistris gel, 10 %; F= Anti-p 120, tris/tricine system, 16 %; G = Anti-p 120, tris/tricine system, 10 %; H = Anti-p 120, tris/glycine system, 12 %; I = Anti-p 120, tris/glycine system, 8 %; J = Anti-p 120, Bistris gel, 10 %. Values indicated above the blots are µg of total protein loaded.
**Figure 11** depicts carrying out a semi-dry Western-Blot using guanidinium hydrochloride containing samples, where the separated samples are electro-transferred to a nitrocellulose membrane.

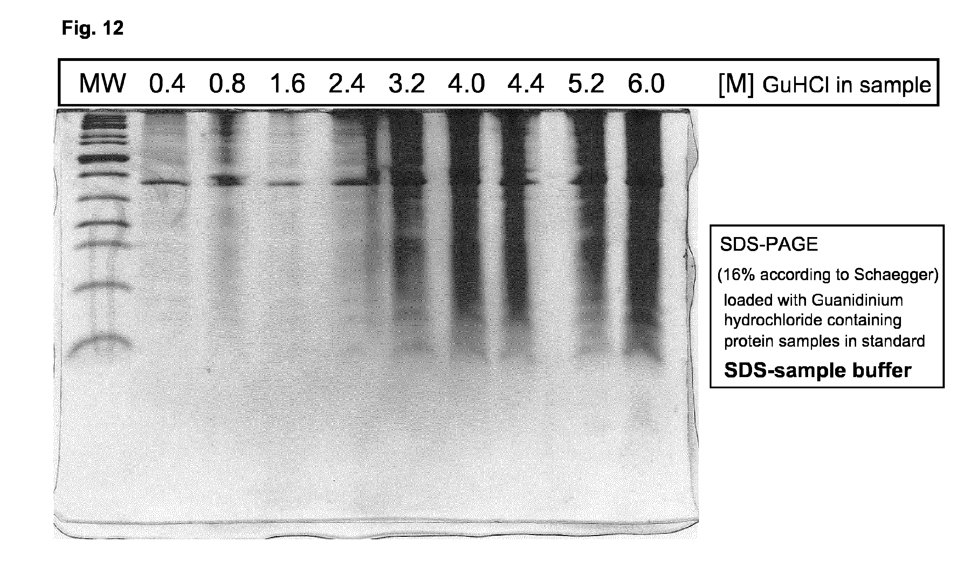

**Figure 12** illustrates electrophoretic separation of guanidinium hydrochloride containing protein samples using tris/tricine SDS-PAGE (according to Schaegger). Bacterial extracts containing 6M guanidinum hydrochloride were diluted to the indicated concentrations and were loaded with SDS-sample buffer.

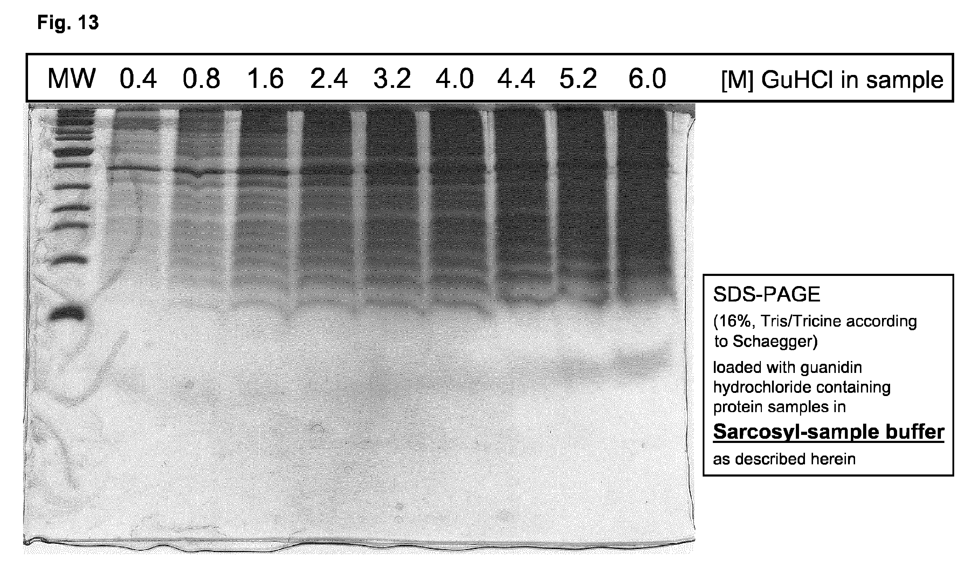

**Figure 13** illustrates electrophoretic separation of guanidinium hydrochloride containing protein samples using tris/tricine PAGE (as disclosed herein). Bacterial extracts containing 6M guanidinum hydrochloride were diluted to the indicated concentrations and were loaded with N-Lauroylsarcosine-containing sample buffer.

### DETAILED DESCRIPTION OF THE INVENTION

Provided in the present disclosure are, amongst others, methods of electrophoretically analyzing a sample, which contains a guanidinium salt, a potassium salt and/or a chaotropic agent. The present invention is at least in part based on the surprising finding that a sample that contains a chaotropic agent, e.g. a guanidinium salt can be electrophoretically separated without removing the chaotropic agent, and nevertheless obtaining separation results that are comparable to conventional electrophoretic separation where samples are used that are devoid of a chaotropic agent. The inventors have found the same result for any guanidinium salt and also for potassium salts. Unexpectedly such electrophoretic separation of a sample that includes a chaotropic agent can be achieved by including N-Lauroylsarcosine into the sample before the sample is exposed to electrophoresis, e.g. gel electrophoresis, including SDS/LDS-PAGE and SDS/LDS-agarose gel electrophoresis. In addition, compared to conventional SDS-PAGE, LDS-PAGE or respective agarose gel electrophoretic separation, SDS, LDS or any other anionic detergent, respectively, used in the sample buffer, may be replaced by N-Lauroylsarcosine. Thus in some embodiments the sample buffer used is essentially, including entirely, void of an alkyl sulfate compound. In one embodiment the sample buffer used is essentially, including entirely, void of SDS or LDS. In one embodiment the sample buffer used contains N-Lauroylsarcosine and furthermore a detergent such as SDS or LDS.

As noted above, the chaotropic agent is in some embodiments a guanidinium salt. Without the intention of being bound by theory it is believed that the inventors' findings show at least in part that the presence of guanidinium salts in a sample interferes with SDS or LDS, thereby causing poor resolution in PAGE (cf. e.g. Fig. 12 and Fig. 13). One of the detergents SDS and LDS is generally added to a sample according to conventional SDS or LDS-PAGE. When SDS or LDS-PAGE is performed, it is customary in the art to also include the respective detergent in the gel buffer and/or the electrophoresis running buffer. It is speculated that chaotropic agents, and in particular guanidinium salts, form precipitates that interfere with electrophoretic sample separation. In this regard activity coefficients, viscosity and surface tension of guanidinium chloride, guanidinium bromide, guanidinium acetate, and guanidinium chloride have been described by Kumar (Fluid Phase Equilibria [2001] 180, 195-204).

### Definitions

Unless otherwise stated, the following terms used in this document, including the description and claims, have the definitions given below.

The word "about" as used herein refers to a value being within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects according to the invention. In this context "about" may refer to a range above and/or below of up to 10%. The word "about" refers in some embodiments to a range above and/or below a certain value that is up to 5%, such as up to up to 2% above or below that value. In some embodiments "about" refers to a range up to 1% above or below a defined value. In some embodiments "about" refers to a range up to 0.5 % above or below that value. In one embodiment "about" refers to a range up to 0.1 % above and below a given value.

The word "assay" as used in this document refers to a method, generally known in the art, to analyze the presence, a feature, e.g. a catalytic activity, the formation or the amount of matter occurring in a biological specimen. Such matter may be occurring in a living organism or representing a living organism, such as a protein, a nucleic acid, a lipid, a cell, a virus, a saccharide, a polysaccharide, a vitamin or an ion, to name a few examples. The word "assay" emphasizes that a certain procedure or series of procedures is followed, which may be taken to represent the respective assay. An assay may include quantitated reagents and established protocols to assess the presence, absence, amount or activity of a biological entity.

The term "analysis" as used herein means both quantitative analysis (e.g., in order to determine the amount of a protein in a sample) as well as qualitative analysis (e.g., in order to determine the molecular weight of a protein in a sample or the determination or identification of novel diagnostic markers for a disease, such as a tumorous disease) detect the existence of specific proteins in an extract, identification of the composition of proteins in a mixture, determining the concentration in the sense of protein content or of specific proteins, to determine the purity of a protein sample, to prove the identity of proteins included in a sample as defined herein. This analysis is carried out by electrophoretic separation of proteins using a gel in gel electrophoresis or a liquid in free flow electrophoresis. In embodiments where gel electrophoresis is being carried out, for instance a polyacrylamide gel or an agarose gel may be used, for example under denaturing conditions, and typically under reducing conditions. The term also includes preparative methods, in which proteins separated by gel electrophoresis are isolated from the gel, or where free flow electrophoresis is carried out that includes collecting the separated components of the sample in a liquid in tubes or a multi well plate.

The term "chaotropic agent" as used herein refers to a compound that is able to disrupt hydrogen bonds of water. Thereby a chaotropic agent is able to disrupt the ordered structure of water molecules. This effect also concerns those water molecules defining a shell around a protein, nucleic acid or polysaccharide. Sample/sample and sample/solvent interactions can thus be reduced. Hydrogen bonding is highly important for the secondary and higher structures of biologic (organic) polymers such as DNA, RNA, and proteins, as well as for the solubility of a molecule in aqueous media. A chaotropic agent can thus be taken as allowing macromolecules more structural freedom and encouraging protein extension and denaturation. Accordingly, a chaotropic agent may also be termed a denaturant. Examples of a chaotropic agent include, but are not limited to, urea, guanidinium salts, tetramethylammonium chloride, guanidine thiocyanate, thiourea, lithium perchlorate, and sodium thiocyanate. In one embodiment the chaotropic agent is or includes urea. In one embodiment the chaotropic agent is urea.In one embodiment the chaotropic agent is different from urea. In one embodiment the chaotropic agent is or includes a guanidinium salt.

A "chaotropic agent", including a "chaotropic salt", is typically a molecule such as a salt that is able to facilitate solubilization of other molecules, which contain apolar moieties, in a polar solvent. A chaotropic salt may for example be a salt that has an anion and/or a cation (as in the case of GuHCl), which assists the interaction and thereby solubilisation of apolar moieties to water. Such salts may, for example, contain the thiocyanate ion, a halide ion such as iodide and bromide, or a hypohalite ion such as perchlorate, as well as a cation such as, for example, lithium, calcium and barium. Exemplary chaotropic salts useful in the practice include sodium thiocyanate, and potassium thiocyanate. Further examples of commonly used chaotropic salts are sodium iodide, and potassium iodide. Examples of chaotropic salts used in the art further include sodium hypochlorite, lithium chlorite, lithium bromide, guanidinium hydrochloride, and guanidinium thiocyanate. In some embodiments the chaotropic agent is a high molecular chaotropic agent, such as a salt containing the guanidinium anion. In some embodiments the chaotropic agent is guanidinium hydrochloride. Two further illustrative examples of a chaotropic salt include guanidinium thiocyanate and guanidinium sulphate. In some embodiments the chaotropic agent is guanidinium bromide. The chaotropic agent may also be guanidinium acetate. In some embodiments the chaotropic agent is guanidinium trifluoroacetate. The chaotropic agent is in some embodiments guanidinioum chlorate. In some embodiments the chaotropic agent is guanidinium nitrate. The chaotropic agent may also be guanidinium carbonate. In some embodiments the chaotropic agent is guanidinium phosphate. The chaotropic agent is in some embodiments guanidinioum hexafluorophosphate. The chaotropic agent may also be guanidinium tetrafluoroborate.

As known by those skilled in the art, such chaotropic agents or salts are commonly used for disruption of the structure of, and denaturing, macromolecules such as proteins and nucleic acids (e.g., DNA and RNA). Chaotropic solutes increase the entropy of the system by interfering with intermolecular as well as intramolecular interactions mediated by non-covalent forces such as hydrogen bonds, van der Waals forces, and hydrophobic effects. Macromolecular structure and function is dependent on the net effect of these forces, therefore it follows that an increase in chaotropic solutes in a biological system will denature macromolecules, reduce or eliminate enzymatic activity and induce stress on a cell. Secondary and tertiary protein folding is dependent (amongst other forces) on hydrophobic forces from amino acids throughout the sequence of the protein. Chaotropic solutes decrease the net hydrophobic effect of hydrophobic regions because of a disordering of water molecules adjacent to the protein. This solubilises the hydrophobic region in the aqueous solution, thereby denaturing the protein. This is also directly applicable to the hydrophobic region in lipid bilayers e.g. a cellular membrane, in an aqueous ambience; if a critical concentration of a chaotropic solute is reached (in the hydrophobic region of the bilayer) then membrane integrity will be compromised, and the cell will lyse. Chaotropic compounds, and chaotropic salts that dissociate in solution exert chaotropic effects via different mechanisms. While non-ionic compounds, such as ethanol, interfere with non-covalent intramolecular forces; salts can in addition have chaotropic properties, by shielding charges and preventing the stabilization of salt bridges. Hydrogen bonding is stronger in non-polar media, so salts, which increase the chemical polarity of the solvent, can also destabilize hydrogen bonding. Mechanistically this is because there are insufficient water molecules to effectively solvate the ions. This can result in ion-dipole interactions between the salts and hydrogen bonding species which are more favourable than normal hydrogen bonds. In addition, solubilization of proteins and protein aggregates in an aqueous solvent containing a high concentration of chaotropic agents generates random coil structure of the protein where hydrophobic amino acid stretches are exposed to the solvent.

A chaotropic agent is generally capable of improving solubility of (insufficient polar) proteins in aqueous medium). The capability of a compound, in particular a salt, to precipitate proteins has been classified in the so called "Hofmeister series". In one embodiment the chaotropic agent as referred to herein is guanidinium hydrochloride (CAS no. 50-01-1). Guanidine hydrochloride is the hydrochloride salt of guanidine. Guanidinium hydrochloride is one of the strongest denaturants used in physiochemical studies of protein folding. In 6 molar (M) guanidinium hydrochloride solution, generally all proteins lose their well ordered structure, and most of them become randomly coiled, i.e. they do not contain any residual secondary and tertiary structure.

The term "denaturation" or "denatured" as used herein means in its broadest sense a process in which proteins lose their tertiary structure and secondary structure by application of some external stress (e.g. sheer stress or heat) or compound (e.g., a denaturant such as a chaotropic salt or agent), or a strong acid or base, a concentrated inorganic salt, an organic solvent (e.g. alcohol or chloroform), a reducing agent. Denatured proteins can exhibit a wide range of characteristics, from high solubility to loss of solubility and communal aggregation. Communal aggregation is the phenomenon of aggregation of the hydrophobic proteins to come closer and form the bonding between them, so as to reduce the total area exposed to water. When a protein is denatured, the secondary and tertiary structures are altered but the peptide bonds of the primary structure between the amino acids are generally left intact. Since all structural levels of the protein usually determine its function/biological activity, the protein can no longer perform its function once it has been denatured. This is in contrast to intrinsically unstructured proteins, which are unfolded in their native state, but still functionally active. In quaternary structure denaturation, protein sub-units are dissociated and/or the spatial arrangement of protein subunits is disrupted. Tertiary structure denaturation involves loss of the proteins three-dimensional structure and arrangement by the disruption of: e.g. covalent interactions between amino acid side-chains (such as disulfide bridges between cysteine groups); non-covalent dipole-dipole interactions between polar amino acid side-chains (and the surrounding solvent); van der Waals (induced dipole) interactions between nonpolar amino acid side-chains. In secondary structure denaturation, proteins lose all regular repeating patterns in more local segments, such as alpha-helices and beta-pleated sheets, and adopt a random coil configuration. Primary structure, such as the sequence of amino acids held together by covalent peptide bonds, is not disrupted by denaturation. Most biological substrates lose their biological function/activity when denatured. For example, enzymes lose their biological activity, because the substrates can no longer bind to the active site of the enzyme, and because amino acid residues involved in stabilizing substrates' transition states are no longer positioned to be able to do so. The denaturing process and the associated loss of activity can be measured using techniques such as dual polarization interferometry, CD, and QCMD. In many proteins, denaturation is reversible which means that the proteins can regain their native state when the denaturing influence is removed. This process can be referred to as renaturation. This understanding has led to the notion that all the information needed for proteins to assume their native state was encoded in the primary structure of the protein, and hence in the DNA that codes for the protein. Accordingly, the term "renaturation", as used herein, refers to the conversion of denatured proteins to a conformation which the proteins have in their native conformations. The term "in vitro renaturation", as used herein, refers to the renaturation which is performed outside the organism.

A "denaturant" as used herein may include, for example, an acid, a solvent, a cross-linking reagent, a chaotropic agent such as a chaotropic salt, a disulfide bond reducer or picric acid. Acidic protein denaturants include, but are not limited to, acetic acid, trichloroacetic acid, for instance 12% in water, and sulfosalicylic acid. Most organic solvents are denaturing, including, for example, ethanol or methanol. Examples of a cross-linking agent for proteins include, for instance, formaldehyde or glutaraldehyde. Chaotropic agents include, e.g., all guanidinium salts such as guanidinium hydrochloride (for example at 6 M) or other chaotropic salts such as lithium perchlorate (at e.g. 4.5 M). Agents that break disulfide bonds by reduction, i.e. reducing agents, include, for example, 2-mercaptoethanol, dithiothreitol (DTT) or TCEP (tris(2-carboxyethyl)phosphine).

The term "non-denaturing conditions" used herein refers to conditions such that denaturation of the desired protein does not occur. These conditions refer to e.g., conditions where no denaturant is present or is present below denaturing concentrations. The term "native conditions" used herein refers to conditions in which the protein molecule can preserve the native conformation and the biological activity.

The term "denaturing electrophoresis" refers to electrophoresis that is being performed under conditions under which the natural structure of the molecule(s) to be analyzed is lost. In electrophoresis a positively or negatively charged molecule is exposed to an electric field. According to its charge, size and shape, a respective molecule migrates in the electric field at a certain speed. Where the molecule(s) of interest are one or more proteins, denaturing electrophoresis means selecting electrophoresis conditions where the protein(s) can be taken to be unfolded, since their native structure is disrupted. Under these conditions the native shape of the respective molecule(s) is essentially without effect on its/their electrophoretic mobility, so that the mass-to-charge ratio can be assumed to be the determining factor for electrophoretic mobility. In the art denaturing conditions during electrophoresis are typically achieved by adding one or more of a denaturant, such as SDS, LDS or urea; a reducing agent; or a combination of both supplements, to the electrophoresis sample buffer and/or - in the case of gel electrophoresis - the running buffer and/or the gel buffer. Typically, the sample of interest is also exposed to elevated temperature conditions. In contrast thereto, in native electrophoresis the natural structure of a molecule to be analyzed is maintained.

The term "direct" as used herein means that components such as proteins of a sample which are resolved in a buffer that contains a chaotropic agent, e.g. guandinium hydrochloride, can be immediately used in electrophoresis such as SDS-polyacrylamide gel electrophoretic analysis, i.e. without the need of an intervening purification and/or precipitation step and/or washing step in order to remove the chaotropic agent, prior to electrophoresis. For example, methods for purification of guanidinium hydrochloride-containing protein samples described in the art include protein precipitation, e.g., by using alcohol, trichloroacetic acid or acetone and subsequent addition to SDS-compatible buffers, separation of the salts by using ion exchange chromatography or other chromatographic methods, by dialysis or by very high dilutions of the samples. Such systems for purification are also commercially available.

The term "essentially consists of" is understood to allow the presence of additional components in a sample or a composition that do not affect the properties of the sample or a composition. As an illustrative example, a pharmaceutical composition may include excipients if it essentially consists of an active ingredient.

A "reducing agent" is a compound or a group of compounds. In particular, a reducing agent is generally a compound that breaks disulfide bonds by reduction, thereby overcoming those tertiary protein foldings and quaternary protein structures (oligomeric subunits) which are stablilized by disulfide bonds. Examples of a suitable reducing agent include, but are not limited to, 2-mercaptoethanol, dithiothreitol (DTT), TCEP (tris(2-carboxyethyl)phosphine), dithioerythritol (DTE), sodium bisulfite, reduced glutathione, cysteamine, tributylphosphine (TBP), dithioerythriol, 2-mercaptoethylamine, dithiobutylamine (DTBA), cysteine or hydroxyethyldisulphide (HED), the latter being commercially available e.g. as "DeStreak Reagent". The use of such reducing agent, mostly in combination with SDS and heating of the sample, is a known procedure for reducing samples for SDS-PAGE. In some embodiments the reducing agent in the sample buffer disclosed herein is DTT. DTT may for instance be present in a concentration of about 20 mM to about 200 mM or more in the sample buffer. In one embodiment about 100mM DTT is used as a concentration in the electrophoresis sample buffer. DTT may be included in the sample in a final concentration of about 6 mM to about 300 mM, before the sample is being loaded onto the gel or inserted into the separation buffer. In some embodiments where gel electrophoresis is being performed a reducing agent added to the running buffer is sodium bisulfite. Sodium bisulfite may for instance be present in a final concentration of about 0.5 mM to about 2 mM in the running buffer. In some embodiments the running buffer may contain more than 2 mM sodium bisulfite. As indicated above, concentrations and amounts indicated with regard to a sample buffer refer to the final concentration of the sample to be applied to electrophoresis, i.e. sample buffer that includes the sample.

A chelating agent is a compound or a group of compounds. Examples of a compound suitable as a chelating agent include, but are not limited to, EDTA, EGTA, diethylenetriaminepentaacetic acid (DTPA), nitrilotriacetic acid (N,N-bis(carboxymethyl)glycine, NTA), diaminocyclohexanetetraacetic acid (DCTA), cyclohexane-1,2-diaminotetraacetic acid (CDTA), diethylenetriamine pentaacetate (DTPA), diethyldithiocarbamate (DEDTC), 1,2-bis(o-amino-phenoxy)ethane-N,N,N',N'-tetraacetic acid (BAPTA), glutamic acid diacetic acid (GLDA) and tripolyphosphate (TPP) or hydroxy-2-ethylenediaminetriacetic acid (HEEDTA). Ethylenediaminetetraacetic acid, widely abbreviated as EDTA, is a polyamino carboxylic acid and a colourless, water-soluble solid. Its conjugate base is named ethylenediaminetetraacetate. Its usefulness arises because of its role as a hexadentate ("six-toothed") ligand and chelating agent, i.e. its ability to "sequester" a metal ion such as Ca²⁺, Mg²⁺ and Fe³⁺ by several coordinate bonds. EDTA is produced as several salts, notably disodium EDTA and calcium disodium EDTA. EGTA (ethylene glycol tetraacetic acid) is a polyamino carboxylic acid, a chelating agent that is related to the better known EDTA, but with a much higher affinity for calcium than for magnesium ions. It is useful for making buffer solutions that resemble the environment inside living cells. In some embodiments the chelating agent in the sample buffer is EDTA. In one embodiment the chelating agent in the sample buffer and in the running buffer is EDTA. As an illustrative example, EDTA may be used in a concentration in the range from about 0.1 mM to about 10 mM in the sample buffer. As an illustrative example, about 3 mM EDTA may be used in the electrophoresis sample buffer. EDTA may be included in the sample in a final concentration in the range from about 0.1 mM or more (to about 8 mM), before the sample is applied to electrophoretic separation. In some embodiments a chelating agent added to the running buffer is EDTA. Where the electrophoresis technique is gel electrophoresis, EDTA may for instance be present in a final concentration of about 0.5 mM to about 5 mM in the running buffer. As an illustrative example, about 1.5 mM EDTA may be used in the gel electrophoresis running buffer. In some embodiments the running buffer may contain more than 5 mM EDTA.

The term "loading aid additive" as used herein refers to an additive that increases the density of the sample, thereby providing an aid for the loading of the sample to the gel in case of carrying out gel electrophoresis. Most SDS-PAGE systems are loaded from the top into loading slots within the gel. To ensure that the sample sinks to the bottom of the gel loading slot, sample buffer is supplemented with additives which are in some embodiments non-ionic and non-reactive towards proteins to avoid interfering with electrophoresis. Common additives are glycerol, sucrose and (synthetic) polysaccharides, e.g. Ficoll (trademark GE healthcare). In some embodiments the loading aid in the sample buffer is glycerol. Glycerol may for instance be used in a final concentration of about 3 % to about 30%. In some embodiments glycerol may be used in a final concentration of about 5 % to about 25%. In some embodiments, about 20% glycerol is used as a final concentration in the gel electrophoresis sample buffer.

The term "polypeptide" or "protein" as used herein means a polymer of amino acids linked by peptide bonds. Thus, for example, the terms peptide, oligopeptide, protein, and enzyme are included within the definition of polypeptide. The polypeptide may be derived, e.g., from a prokaryotic organism, such as bacteria or archaea, or a eukaryotic organism, including, for example, mammals. Accordingly, the protein can be a prokaryotic or eukaryotic protein, a recombinant or a (isolated) naturally occurring protein.The polypeptide may also be a viral protein. The mentioned term also includes post-expression modifications of the polypeptide, such as glycosylations, acetylations, phosphorylations. The term polypeptide does not connote a specific length of a polymer of amino acids. Where both terms "polypeptide" and "protein" are used concurrently, this twofold naming accounts for the use of both terms side by side in the art. A polypeptide may be isolatable directly from a natural source, or can be prepared with the aid of recombinant, enzymatic, *in-vitro* synthesis, or (bio)chemical techniques. In the case of a polypeptide that is naturally occurring, such a polypeptide is typically isolated. An "isolated" polypeptide is one that has been removed from its environment. For instance, an isolated polypeptide is a polypeptide that has been removed from the cytoplasm or from the membrane of a cell, and many of the polypeptides, nucleic acids, and other material of its environment are no longer present. An "isolatable" polypeptide is a polypeptide that could be isolated from a particular source. A "purified" polypeptide is one that is at least 60% free, at least 75% free, and in some embodiments at least 90% free from other components with which they are associated. As used herein, a "polypeptide fragment" refers to a portion of a polypeptide that results, e.g., from digestion of a polypeptide with a protease. Such fragments may have a length of about 30 or more amino acids. In some embodiments a polypeptide fragment has a length of 40 or more amino acids, including about 50 or more amino acids. A polypeptide fragment may in some embodiments have a length of 75 amino acids or more. In some embodiments a polypeptide fragment has a length of about 100, 150, 200, 250, 300, 400, 500 or even more amino acid residues.

A polypeptide as used herein may be characterized by molecular mass, mass fingerprint, or the combination thereof. The molecular mass of a polypeptide, typically expressed in kilodaltons (kDa), can be determined using routine methods including, for instance, gel filtration, gel electrophoresis including SDS-PAGE, capillary electrophoresis, mass spectrometry, and liquid chromatography including HPLC. For example, the molecular mass of a polypeptide can be determined by resolving polypeptide using a polyacrylamide gel, such as an SDS polyacrylamide gel having a stacking gel of about 4% and a resolving gel of about 10% acrylamide under reducing and denaturing conditions. Unless indicated otherwise, molecular weight refers to molecular mass as determined by gel electrophoresis such as SDS-PAGE. As used herein, a "mass fingerprint" refers to a population of polypeptide fragments obtained from a polypeptide after digestion with a protease. Typically, the polypeptide fragments resulting from a digestion are analyzed using a mass spectrometric method. Each polypeptide fragment is characterized by a mass, or by a mass (m) to charge (z) ratio, which is referred to as an "m/z ratio" or an "m/z value". Methods for generating a mass fingerprint of a polypeptide are routine.

The term "inclusion body" as used herein means a cell nucleus or cytoplasmic aggregate of stainable substances, usually proteins. More specifically, the inclusion bodies as used herein typically mean insoluble or only slightly soluble aggregates of recombinant proteins which may contain in addition other molecules, formed upon expression in microbes or bacteria, such as *Escherichia coli (E. coli*). The proteins in the inclusion bodies are thought to contain predominantly misfolded proteins and are well described in the art.

A "running buffer" is an aqueous solution of a buffer compound, typically containing one or more further components, which is used to provide a contact between two ends of an electrophoresis gel, an anode and a cathode. A running buffer is not mandatory for carrying out electrophoresis, but it is included in the standard systems used in the art. Therefore in many embodiments of a method disclosed herein a running buffer is used. In some embodiments the same running buffer is used to connect the anode and the electrophoresis gel and the cathode and the electrophoresis gel. A running buffer may include two different buffers, of which one contacts the cathode and one the anode during electrophoresis. The running buffer is known to serve a plurality of purposes, such as maintaining a desired pH, dissipating heat, and providing a fluid communication between anode and a cathode via the electrophoresis gel, so that an electric field can be generated. Where a detergent is used in the electrophoresis system, such as SDS or LDS, the running buffer may also serve as a continuous supply of the detergent for the electrophoresis gel.

A "sample" may include any matter. It is understood that electrophoretic separation, including analysis, using an electrophoresis gel can only be performed on matter that is soluble in the desired buffer system to be used. On the other hand, particles of inorganic or organic nature, including cells (see e.g. Weber, G., et al. Electrophoresis [2000] 21, 325-328; or Dong, Y.-C., et al., J. Sep. Sci. [2001] 34, 1683-1691), viruses, organelles or liposomes, may be separated by electrophoresis using a gel-free system in free-flow electrophoresis. Nevertheless, also for gel electrophoresis the sample may include for instance solid matter that is insoluble in the desired sample buffer selected for use. In gel electrophoresis such solid matter will usually be very restricted in the ability to enter the gel and therefore preliminary be separated from soluble matter due to this fact. Depending on size, shape and surface charge very small particular matter, typically of a width below a micrometer such as nanomaterial, may also migrate and separate in gel electrophoresis (Kim et al., J.-Y, Electrophoresis [2013] 34, 6, 911-916; see also Guarrotxena, N., and Braun, G., J. Nanopart. Res. [2012] 14, 1199). Likewise, while the sample may contain low molecular weight compounds, these compounds will typically not be detected in electrophoretic analysis using conventional techniques - albeit a separation may occur. Typical examples of suitable samples commonly used in the art for electrophoretic analysis - both in gel electrophoresis and free-flow electrophoresis, include, but are not limited to a sample containing a peptide/protein, a nucleic acid, a lipid, a polysaccharide. The sample may be of any origin. It may for instance, but not limited to, be derived from humans, animals, plants, bacteria, viruses, spores, fungi, or protozoae, or from organic or inorganic materials of synthetic or biological origin. Accordingly, any of the following samples selected from, but not limited to, the group consisting of a soil sample, an air sample, an environmental sample, a cell culture sample, a bone marrow sample, a rainfall sample, a fallout sample, a sewage sample, a ground water sample, an abrasion sample, an archaeological sample, a food sample, a blood sample, a serum sample, a plasma sample, an urine sample, a stool sample, a semen sample, a lymphatic fluid sample, a cerebrospinal fluid sample, a nasopharyngeal wash sample, a sputum sample, a mouth swab sample, a throat swab sample, a nasal swab sample, a bronchoalveolar lavage sample, a bronchial secretion sample, a milk sample, an amniotic fluid sample, a biopsy sample, a cancer sample, a tumour sample, a tissue sample, a cell sample, a cell culture sample, a cell lysate sample, a virus culture sample, a nail sample, a hair sample, a skin sample, a forensic sample, an infection sample, a nosocomial infection sample, a production sample, a drug preparation sample, a biological molecule production sample, a protein preparation sample, a lipid preparation sample, a carbohydrate preparation sample, an engineered sample, a space sample, an extraterrestrial sample or any combination thereof may be processed in the method. Where desired, a respective sample may have been preprocessed to any degree. As an illustrative example, a tissue sample may have been digested, homogenized or centrifuged prior to electrophoretic analysis. The sample may furthermore have been prepared in form of a liquid, such as a solution. Examples include, but are not limited to, a solution or a slurry of a nucleotide, a polynucleotide, a nucleic acid, a peptide, a polypeptide, an amino acid, a protein, a synthetic polymer, a biochemical composition, an organic chemical composition, a lipid, a carbohydrate, a combinatory chemistry product, a drug candidate molecule, a drug molecule, a drug metabolite or of any combinations thereof. Further examples include, but are not limited to, a suspension of a cell, one or more subcellular particles or organelles, a virus, a microorganism, a pathogen, a radioactive compound or of any combinations thereof. It is understood that a sample may furthermore include any combination of the aforementioned examples.

The term "protein sample" as used herein means a sample containing or one or more proteins or polypeptides as defined herein. Protein samples as used herein may be any material containing peptides or proteins, such as, for example, prokaryotic or eukaryotic cells, cellular components such as subcellular particles or organelles, cell debris, membranes, tissues, organs, body fluids, environmental samples such as environmental particles, engineered materials and particles such as engineered nanomaterials, viruses, cell culture supernatants containing released proteins or other materials, or purified or isolated naturally occurring proteins as well as recombinant, or (bio)chemically or in vitro synthesized peptides, including proteins. In the case of solid tissues, these are often first broken down chemically or mechanically using a blender (for larger sample volumes), a homogenizer (smaller volumes), or a sonicator or by using high pressure or repeated freeze-thaw cycles. Cells may also be broken open by one or several of the above mechanical methods, by enzymatic digestion or by cell lysis buffers known in the art. In the case of tissues or cells, a combination of biochemical and mechanical techniques - including various types of filtration and centrifugation - may be used to separate different cell compartments and organelles prior to electrophoresis. Such methods are also described in the art. Samples of body fluids can be obtained by well known techniques and include, for example, samples of blood, plasma, serum, or urine. Tissue or organ samples may be obtained from any organism and any tissue or organ by, e.g., biopsy. Cells may be obtained from the body fluids or the tissues or organs by separating techniques such as centrifugation, filtration or cell sorting (FACS). The cells can be cultured cells or primary cells from any organism and any tissue or organ. In some embodiments the sample as used herein contains at least one polypeptide as defined herein and at least one denaturant such as a chaotropic agent as set forth herein. The protein sample can include more than one denaturant such as two, three or even more chaotropic agents.

A "sample buffer" as used herein refers to one or more compounds, typically one or more solutions, that is/are added to a sample to be analyzed prior to electrophoretic analysis (cf. also below). It is understood that compounds such as N-Lauroylsarcosine, a buffer compound or possibly a reducing agent may be added to the sample individually. It has, however, become standard practice in the art to combine all additives that are to be added to the sample prior to electrophoretic analysis in a buffered solution, and to refer to this solution as a sample buffer. Typically, the sample buffer is used as a sample buffer concentrate, which is being diluted to the final concentrations of buffer and additives as a consequence of the addition to the sample. To facilitate reading, throughout this description, reference is made to a sample buffer wherever compounds are being added to a sample before analyzing the same by electrophoresis - regardless of whether a single solution containing a plurality of compounds, individual compounds, individual solutions of compounds or a combination of any of these are used.

SDS-polyacrylamide gel electrophoretic analysis of proteins is well described in the art. Accordingly, the term "SDS-polyacrylamide gel electrophoretic analysis (SDS-PAGE)" or "sodium dodecyl sulphate polyacrylamide gel electrophoresis" as used herein describes a collection of related techniques widely used in biochemistry, forensics, genetics and molecular biology to separate proteins according to their electrophoretic mobility, which is a function of the length of a polypeptide chain and its charge. In most proteins, the binding of SDS to the polypeptide chain imparts an even distribution of charge per unit mass, thereby resulting in a fractionation by approximate size during electrophoresis. Means and methods for carrying out SDS-PAGE are further defined in the following.

The term "tracking dye" as used herein means a dye which can be used to follow the progress of the protein sample during electrophoresis, for example through the gel in case of gel electrophoresis. Proteins are mostly colourless which means that their migration, through the gel or the separation buffer, during electrophoresis cannot be easily followed. Anionic dyes of a known electrophoretic mobility are, therefore, usually included in the electrophoresis sample buffer. A very common tracking dye is Bromophenol blue (BPB, 3',3",5',5" tetrabromophenolsulfonphthalein). This dye is coloured at alkali and neutral pH and is a small negatively charged molecule that moves towards the anode. Being a highly mobile molecule it moves ahead of most proteins. As it reaches the anodic end of the electrophoresis medium electrophoresis is stopped. It can weakly bind to some proteins and impart a blue colour. Another example for a tracking dye is Coomassie Brilliant Blue (CBB). "Coomassie Brilliant Blue R-250 ("red blue")" (C45H44N3NaO7S2; mW: 825.97) or "Coomassie G-250" ("blue silver") as used herein is an anionic dye, which non-specifically binds to proteins. The structure of CBB is predominantly non-polar, and it is usually used in methanolic solution acidified with acetic acid. For visualisation after electrophoresis, proteins in a gel are fixed by alcohol and/or acetic acid and simultaneously stained. The excess dye incorporated into the gel can be removed by de-staining with the same solution without the dye. The proteins are detected as blue bands on a clear background. As SDS is also anionic, it may interfere with staining process. Therefore, preceding washing procedures and large volume of staining solution may be recommended in such cases, at least ten times the volume of the gel. A further example of a tracking dye is phenol red. In some embodiments the tracking dye in the sample buffer is BPB. BPB may for instance be used in a final concentration of about 0.0005 % to about 0.003 %. In some embodiments about 0.001% BPB or more is used as a final concentration in the electrophoresis sample buffer.

The terms "comprising", "including," containing", "having" etc. shall be read expansively or open-ended and without limitation. Singular forms such as "a", "an" or "the" include plural references unless the context clearly indicates otherwise. Thus, for example, reference to a "protein" includes a single protein as well as a plurality of proteins. Likewise reference to a "cell" includes a single cell as well as a plurality of cells. Unless otherwise indicated, the term "at least" preceding a series of elements is to be understood to refer to every element in the series. The terms "at least one" and "at least one of" include for example, one, two, three, four, or five or more elements. It is furthermore understood that slight variations above and below a stated range can be used to achieve substantially the same results as a value within the range. Also, unless indicated otherwise, the disclosure of ranges is intended as a continuous range including every value between the minimum and maximum values.

The scope and meaning of any use of a term will be apparent from the specific context in which the term is used. Certain further definitions for selected terms used throughout this document are given in the appropriate context of the detailed description, as applicable. Unless otherwise defined, all other scientific and technical terms used in the description, figures and claims have their ordinary meaning as commonly understood by one of ordinary skill in the art.

### ELECTROPHORETIC SEPARATION OF SAMPLES CONTAINING A CHAOTROPIC AGENT

Thus far, where a chaotropic agent is present in a sample that is to be analyzed by electrophoresis such as gel electrophoresis, the chaotropic agent needs to be removed or at least be reduced in concentration prior to electrophoretic analysis, such as SDS-PAGE. However, an at least essentially complete removal or partial removal of a chaotropic agent is both laborious and time consuming, particularly if multiple samples have to be handled at the same time. In addition, methods are frequently associated with qualitative or quantitative losses of sample material which can be caused, e.g., by incomplete precipitation, by inefficient resolving precipitated proteins, by incomplete refolding, by precipitation of proteins upon dialysis or ion exchange chromatography, and also by selective loss of particularly hydrophobic proteins, due to non-solvent precipitates and undesired adhesion to material surfaces.

A method as described in this document circumvents these issues by allowing analysis of protein samples without the need to remove the chaotropic agent. In this document the application of a sample to an electrophoresis gel or a free flow electrophoresis separation buffer, without removal of a chaotropic agent included in the sample, is also referred to as "direct analysis" or "direct electrophoresis". In a method described in this disclosure the sample, which includes a chaotropic agent is dissolved in one or more buffers containing one or more chaotropic agents, high molecular weight chaotropic salt solutions, such as a guanidinium salt or a thiocyanate. No removal of the chaotropic agent is necessary any longer, prior to electrophoresis such as SDS-PAGE, as in the approaches used in the prior art thus far. A method disclosed in this document is particularly suitable for protein samples resolved in a buffer containing up to about 6 M, or even up to about 7 M or about 8 M guanidinium hydrochloride.

Advantageously, a method as described in this document has been established for use with any of the various known gel/buffer systems of gel electrophoresis. These systems include SDS-PAGE, e.g., the tris/glycine-SDS-PAGE according to Laemmli (Nature [1970] 227, 680-685; J. Mol. Biol. 80, 575-599), Tis/tricine-SDS-PAGE according to Schaegger (Nature Prot. [2006] 1, 16-22), Bistris-SDS/LDS-PAGE with pH neutral buffer system, adapted according to Graham et al. (Proteomics 2005, 5, 2309-2314) and Kinoshita and Kinoshita-Kikuta (Proteomics [2011] 11, 319-323), CriterionTM gels (BioRad), the Bistris-PAGE mini gel system (NovexTM-Invitrogen) or NuPAGE® Pre-Cast Gel System and others. The method described in this document can be used to any desired gel embodiments, such as for example a slab gel, which may be used in a vertical or horizontal arrangement while an electric field is applied, a tube gel or a flow chamber. Where a slab gel is used, typically continuous zone electrophoresis will be carried out. Where a tube gel is used, typically continuous capillary zone electrophoresis will be carried out.

A further example of suitable electrophoresis gels are agarose gels. An overview of agarose gel electrophoresis, as it may also be employed in a method described in this document, has been given by Warren et al. (Electrophoresis [2003] 24, 1695-1702). A protocol describing how to perform agarose gel electrophoresis on a sample that contains protein has been given by Krizek and Rick (Current Protocols in Cell Biology [2002], chapter 6.7). Wu and Kusukawa (BioTechniques [1998] 24, 4, 676-678) have provided agarose gel electrophoresis using a stacking gel that contains 1 % agarose and has a pH value of 6.8, and a resolving gel containing 7 % agarose and 1 M urea and having a pH value of 8.6. The use of agarose gels for electrophoretic separation of a sample that contains proteins is advantageous for separating proteins that are larger than about 500 kD, in particular proteins larger than about 600 kD, where polyacrylamide gels show poor resolution. An example for a frequently used application of SDS agarose gel electrophoresis is the analysis of very large proteins in human serum samples, such as von-Willebrand-factor multimers, which play a pivotal role in haemostasis and thrombosis, and are associated with prolonged bleedings and other life-threatening diseases (Barg, A., et al., Thromb. Haemost. [2007] 97, 514-526). An agarose gel is prepared by casting a hot solution of agarose powder in a gel buffer into an assembled gel formation set-up. Agarose is a linear galactan polymer, generally consisting of alternating D-galactose and 3,6-anhydro-L-galactose units. The melting point of agarose is, depending on the source of agarose and its concentration, between about 85 and about 95 °C. For convenience it is common to form a respective hot solution by bringing its temperature to the boiling point. Typically the gel formation set-up, into which this a hot solution is to be filled, is prewarmed to a temperature above room temperature. As an illustrative example the gel formation set-up may be brought to a temperature of about 60 or 70 °C. The hot solution of agarose powder in the gel buffer may be allowed to cool to the same temperature range, such as a temperature of about 60 or 70 °C, before casting this solution. Molten agarose solidifies, again depending on the source of agarose and its concentration, in the temperature range from about 32 to about 42 °C. Upon cooling to room temperature the agarose solidifies, forming an electrophoresis gel. Accordingly, the assembled gel formation set-up with the agarose solution filled therein is allowed to cool to room temperature. It is advantageous to maintain the assembled gel formation set-up with the agarose solution filled therein for a period of time such as e.g. 5 minutes or 10 minutes at an elevated temperature such as 60 °C, for instance to allow bubbles to escape and for a homogenous solution to form. An agarose gel can be stained with a conventional dye such as Coomassie^{®} Blue or silver. In some embodiments a gel that contains both agarose and polyacrylamide may be used to carry out electrophoretic sample analysis.

Those skilled in the art will thus appreciate that independent from the choice of gel system or free flow system used, using the method described herein a sample that contains a guanidinium salt, a potassium salt and/or a chaotropic agent may be conveniently analyzed without the need of buffer exchange or removal of the guanidinium salt, potassium salt and/or chaotropic agent. Unexpectedly, and particularly advantageous, a method disclosed herein for electrophoretic gel analysis, can be carried out with the characteristic running and separating properties of the above gel/buffer systems remaining substantially unchanged, as illustrated e.g. in Figures 4, 5, or 7, and the following examples. This is due to the fact that the buffers have been established in a way that they can be used in combination with gels and buffers of gel/buffer systems established in the art. This has been exemplified for the tris/tricine PAGE according to Schaegger (Fig. 4), the tris/glycine PAGE according to Laemmli (Fig. 5), the Criterion™ Bistris PAGE (BioRad Laboratories, Munich, Germany) (Fig. 6) and the neutral Bistris PAGE (Fig. 7), as also demonstrated in the following examples.

The methods described in this document are further particularly useful for the separation and analysis of heterologous proteins or fusion proteins such as tagged proteins (e.g., His-tagged or GST-tagged proteins). In addition, there are provided novel and efficient tools for the separation and analysis of recombinant proteins embedded or included in inclusion bodies and other proteins that are difficult to solubilize in aqueous media. In general, the method is particularly useful for direct analysis and separation of samples that contain proteins in a solution that includes a denaturant, e.g. a chaotropic agent such as a guanidinium salt. A respective sample may for example originate from, include, essentially consist of or consist of an extract from cells or tissue, for example including hydrophobic proteins and/or membrane proteins, or non-soluble precipitates.

The most commonly used form of gel electrophoresis used in the art to analyze samples that contain proteins, is SDS-PAGE. Several protocols have been developed in the art to carry out SDS-PAGE, any of which may be used in a method described herein. In conventional SDS-PAGE analysis, the sample to be analyzed is mixed with SDS, an anionic detergent which denatures secondary and non-disulfide-linked tertiary structures, and applies a negative charge to each protein in proportion to its mass. Heating the samples to at least 50°C further promotes protein denaturation, helping SDS to bind (see, e.g., Laemmli (1970), Nature 227, pages 680 - 685). In the sample buffer of a system as described herein, SDS or LDS has generally been replaced by N-lauroylsarcosine. In the running buffer used, N-lauroylsarcosine is typically used in addition to an anionic detergent such as SDS, LDS or ε-aminocaproic acid. The used gel may also contain such anionic detergent, depending on the type of gel/buffer system used. The combination of N-lauroylsarcosine and anionic detergent may be advantageous to achieve a good separation of proteins on the gel and assists entry of proteins into the gel at the beginning of gel electrophoresis, thought to be due to reduced precipitation.

In a method described herein a detergent may for instance be included in the gel buffer, the sample buffer, in the running buffer and/or a separation buffer. Any detergent contemplated can generally be employed in the method. Any amphiphilic compound that lowers the surface tension of an aqueous solution may be used as a respective detergent. The terms "detergent" and "surfactant" are used synonymously herein. An anionic detergent, a cationic detergent, a zwitterionic detergent or a non-ionic detergent may be employed, depending on the selected underlying protocol.

An example of an anionic detergent is sodium dodecyl sulfate (SDS). SDS (C₁₂H₂₅NaO₄S; mw: 288.38) is a strong anionic detergent used to denature native proteins to unfolded, polypeptides. A further illustrative example of a strong anionic detergent is lithium dodecyl sulfate (LDS; C₁₂H₂₅O₄SLi, mw: 272.3). When a protein mixture is heated to about 70°C to about 100°C in presence of SDS or LDS and a reducing agent, the detergent wraps around the polypeptide backbone. It binds to most polypeptides in a constant weight ratio of 1.4 g SDS/g of polypeptide which gives all SDS-bound proteins roughly the same charge to mass ratio, and hence equal mobilities in an electric field. In this process, the intrinsic charges of polypeptides become negligible when compared to the negative charges contributed by SDS. Thus, polypeptides after treatment become rod-like structures possessing a uniform charge density, that is same net negative charge per unit length. The electrophoretic mobilities of these proteins will be a linear function of the logarithms of their molecular mass. Further examples of an anionic detergent include, but are not limited to, sodium glycocholate (CAS No. 863-57-0), sodium cholate (CAS No. 73163-53-8), and sodium taurocholate (CAS No. 145-42-6). Sodium N-lauroylsarcosine is likewise an anionic detergent. Typically some buffers used in a method described in this specification contain a further detergent.

Three illustrative examples of a cationic detergent are hexadecyltrimethylammonium bromide (cetyltrimethylammonium bromide; CTAB, Chemical Abstracts No. 57-09-0), trimethyl-(tetradecyl)ammonium bromide (TTAB, CAS No. 1119-97-7), and benzyldimethyl-n-hexadecyl-ammonium chloride ("16-BAC"). Examples of a non-ionic detergent include, but are not limited to, [N,N'-bis(3-D-gluconamidopropyl)cholamide] (BigCHAP, CAS No. 86303-22-2), N,N'-bis-(3-D-gluconamidopropyl)deoxycholamide (Deoxy BigCHAP, CAS No. 86303-23-3), Polyoxyethylene monolauryl ether (Brij® 35, Chemical Abstracts No. 9002-92-0), Decyl-β-D-maltopyranoside (CAS No. 82494-09-5), n-Dodecyl-β-Dmaltoside (CAS No. 69227-93-6), poly(propylene glycol) available as Pluronic® F-68 (CAS No. 9003-11-6) and 1,1,3,3-tetramethylbutyl)phenyl-polyethylene glycol, also known as polyethylene glycol tert-octylphenyl ether and Triton® X-114 (CAS No. 9036-19-5). Examples of a zwitter-ionic detergent include, but are not limited to, 3-[(3-cholamidopropyl)dimethylammonio]-1-propanesulfonate (CHAPS, CAS No. 75621-03-3), 3-([3-cholamidopropyl]dimethylammonio)-2-hydroxy-1-propanesulfonate (CHAPSO, CAS No. 82473-24-3), amidosulfobetaine-14 (ASB-14, CAS No. 216667-08-2), n-dodecyl-N,N-dimethylglycine (EMPIGEN® BB, CAS No. 683-10-3), 3-(N,N-dimethyloctylammonio)propanesulfonate inner salt (SB3-8, CAS No. 15178-76-4), 3-(decyldimethylammonio)propanesulfonate inner salt (SB3-10, CAS No. 15163-36-7), and 3-(4-heptyl)phenyl-3-hydroxypropyl)dimethylammoniopropane-sulfonate (3-(4-Heptyl)phenyl-3-hydroxy-propyl-dimethylammonio-sulfobetaine, C7BzO).

In the absence of SDS, different proteins with similar molecular mass would migrate differently, due to differences in mass-charge ratio, as each protein has an isoelectric point and molecular weight specific for its primary structure. This is known as native gel electrophoresis, e.g. native PAGE. Adding SDS solves this problem, as it binds to and unfolds the protein, giving a near uniform negative charge along the length of the polypeptide.

A tracking dye is usually added to the protein solution. This typically has a higher electrophoretic mobility than the proteins to allow tracking the progress of the proteins through the gel during the electrophoretic run.

An electrophoresis gel may in some embodiments be a polyacrylamide gel that includes acrylamide, bisacrylamide, and a buffer with an adjusted pH. In some embodiments the electrophoresis gel further includes SDS. Typically an electrophoresis gel consists of acrylamide, bisacrylamide, SDS, and a buffer with an adjusted pH. The solution may be degassed under a vacuum to prevent the formation of air bubbles during polymerization. A source of free radicals and a stabilizer such as ammonium persulfate and TEMED are added to initiate polymerization. The polymerization reaction results in a gel because of the added bisacrylamide, generally about 1 part in 37.5 relative to acrylamide, which can form cross-links between two polyacrylamide molecules. The ratio of acrylamide to bisacrylamide can be varied for specific purposes. The acrylamide concentration of the gel can also be varied, generally in the range from 3% to 25%. Lower percentage gels are better for resolving very high molecular weight proteins, while much higher percentages are needed to resolve smaller proteins. Determining how much of the various solutions to mix together to make gels of particular acrylamide concentration is known in the art.

Gels are usually cast between two glass plates in a gel caster, or in a casting tray, with a comb inserted at the top to create the sample wells. After the gel is solidified or polymerized the comb can be removed and the gel is ready for electrophoresis.

A method according to the present disclosure can generally by applied to any electrophoresis technique available in the art. Various buffer systems are for instance used in gel electrophoresis such as SDS-PAGE depending on the nature of the sample and the experimental objective. Generally, any such buffer system can be applied in the context of a method described in this specification. The buffers used at the anode and cathode may be the same or different; see, e.g., Schaegger (1987), Anal Biochem 166 (2), pages 368-379. As set forth elsewhere herein, the sample buffer, and in the case of gel electrophoresis the running buffer and the gel buffer are particularly advantageous for the direct analysis of chaotropic agent-containing protein samples, without the need for removal of the chaotropic agent, prior to electrophoresis such as SDS-PAGE. In the methods known in the art, the chaotropic agent had to be eliminated from the protein sample by laborious and time-consuming methods, such as dialysis, TCA precipitation or methanol/chloroform/water precipitation, gel filtration or chromatographic approaches. However, these procedures are frequently associated with loss of protein material which can be avoided by the methods disclosed in this specification.

In gel electrophoresis an electric field is applied across the gel, causing the negatively-charged proteins to migrate across the gel towards the positive (+) electrode (anode). Depending on their size, each protein will move differently through the gel matrix: short proteins will more easily fit through the pores in the gel, while larger ones will have more difficulty since they encounter more resistance. After a set amount of time (usually less than an hour to a few hours - though this depends on the voltage applied across the gel; higher voltages run faster, but tend to produce somewhat poorer resolution), the proteins will have differentially migrated based on their size; smaller proteins will have travelled farther down the gel, while larger ones will have remained closer to the point of origin. Proteins may therefore be separated roughly according to size and, thus, molecular mass; however, as known to those skilled in the art, certain glycoproteins behave anomalously on SDS gels. In a free flow system an electric field is applied across the separation buffer, an aqueous liquid, in a separation chamber (see e.g. Weber, J.A., et al., Current Protocols in Protein Science [2003] Wiley & Sons, Supplement 32, chapter 22.5, for an exemplary protocol). Again, negatively-charged proteins are caused to migrate towards the positive electrode (anode), and positively-charged proteins are caused to migrate towards the negative electrode (cathode). In some embodiments the sample may in addition be exposed to a laminar flow, in which case the electric field is typically applied at least essentially perpendicular to the laminar flow. The components of the sample may be separated for example by their isoelectric point, the pl value (isoelectric focusing), by their net charge (zone electrophoresis), or by their electrophoretic mobility (isotachophoresis). In free flow electrophoresis a detergent may also serve in improving separation quality by stabilizing a continuous analyte stream (Frost, N.W., and Bowser, M.T., Lab on a Chip [2010] 10, 1231-1236).

A method as described in this document is generally compatible with any analysis technique that may be carried out following electrophoretic separation. Following electrophoresis, the gel may be stained, most commonly with Coomassie Brilliant Blue (CBB) R-250 or CBB G-250, or silver stain, allowing visualization of the separated proteins, or processed further, e.g. by techniques to transfer proteins on a solid support such as transfer by Western blotting or used for preparative techniques, such as for example preparative electrophoresis to isolate proteins, or any other application known in the art. After staining, different proteins will appear as distinct bands within the gel. It is common to run molecular mass size markers of known molecular mass in a separate lane in the gel, in order to calibrate the gel and determine the approximate molecular mass of unknown proteins by comparing the distance travelled relative to the marker.

SDS-PAGE is usually the first choice as an assay of protein purity due to its reliability and ease. The presence of SDS or LDS and the denaturing step causes proteins to be separated approximately based on size, but aberrant migration of some proteins may occur. Depending on the staining technique employed, different proteins may also stain differently, which interferes with quantification by staining. SDS-PAGE may also be used as a preparative technique for the purification of proteins. For example, **q**uantitative **p**reparative **n**ative **c**ontinuous **p**oly**a**crylamide **g**el **e**lectrophoresis (QPNC-PAGE) is a method for separating native metalloproteins in complex biological matrices.

A polyacrylamide gel (PAG) is a synthetic, thermo-stable, transparent, strong, chemically relatively inert gel, formed by polymerisation of acrylamide. A respective gel can be prepared with a wide range of average pore sizes. The pore size of a gel is determined by two factors, the total amount of acrylamide present (%T) (T = Total concentration of acrylamide and bisacrylamide monomer) and the amount of cross-linker (%C) (C = bisacrylamide concentration). Pore size decreases with increasing %T; with cross-linking, 5%C gives the smallest pore size. Any increase or decrease in %C from 5% increases the pore size, as pore size with respect to %C is a parabolic function with vertex as 5%C. This appears to be because of non-homogeneous bundling of polymer strands within the gel. This gel material can also withstand high voltage gradients, is amenable to various staining and distaining procedures, and can be digested to extract separated fractions or dried for autoradiography and permanent recording.

The counterion balances the intrinsic charge of the buffer ion and also affects the electric field strength during electrophoresis. Highly charged and mobile ions are often avoided in SDS-PAGE cathode buffers, but may be included in the gel itself, where it migrates ahead of the protein. In applications such as DISC SDS-PAGE, the pH values within the gel may vary to change the average charge of the counterions during the run to improve resolution. Commonly used compounds that are capable of providing a counterion are e.g. glycine or tricine. Glycine has been used as the source of trailing ion or slow ion, because its pKa is 9.69 and mobility of glycinate are such that the effective mobility can be set at a value below that of the slowest known proteins of net negative charge in the pH range. The minimum pH of this range is approximately 8.0.

When dissolved in water, slow, spontaneous autopolymerisation of acrylamide (C₃H₅NO; MW: 71.08) takes place, joining molecules together by head on tail fashion to form long single-chain polymers. The presence of a free radical-generating system greatly accelerates polymerization. This kind of reaction is known as "Vinyl addition polymerization". A solution of these polymer chains becomes viscous but does not form a gel, because the chains simply slide over one another. Gel formation requires linking various chains together. Acrylamide is a neurotoxin. It is also essential to store acrylamide in a cool dark and dry place to reduce autopolymerisation and hydrolysis.

An acrylamide gel is prepared by polymerizing acrylamide in the presence of a cross-linking agent. Bisacrylamide (N,N'-Methylenebisacrylamide) (C₇H₁₀N₂O₂; mW: 154.17) is the most frequently used cross linking agent for polyacrylamide gels. Chemically it can be thought of as two acrylamide molecules coupled head to head at their non-reactive ends. Bisacrylamide can crosslink two polyacrylamide chains to one another, thereby resulting in a gel.

Radicalic polymerisation generally requires the use of an initiator. Ammonium persulfate (APS) (N₂H₈S₂O₈; mW: 228.2) acts a source of free radicals and is often used as an initiator for gel formation. A further source of free radicals is riboflavin, which generated free radicals in a photochemical reaction.

In the art typically TEMED (N,N,N',N'-tetramethylethylenediamine) (C₆H₁₆N₂; mW: 116.21) is used in acrylamide gel polymerisation together with APS. TEMED stabilizes free radicals and improves polymerisation. The rate of polymerisation and the properties of the resulting gel depend on the concentrations of free radicals. Increasing the amount of free radicals results in a decrease in the average polymer chain length, an increase in gel turbidity and a decrease in gel elasticity. Decreasing the amount shows the reverse effect. The lowest catalytic concentrations that will allow polymerisation in a reasonable period of time should be used. APS and TEMED are typically used at approximately equimolar concentrations in the range of 1 to 10 mM.

As noted above, besides the addition of SDS or LDS in conventional SDS-PAGE, proteins may optionally be briefly heated to an elevated temperature, including a temperature at or near the boiling point in the presence of a reducing agent, such as dithiothreitol (DTT), 2-mercapto-ethanol (beta-mercaptoethanol/BME), tris(2-carboxyethyl)phosphine (TCEP), dithioerythritol (DTE), reduced glutathione, cysteamine, tri-n-butylphosphine (TBP), dithioerythriol, 2-mercapto-ethylamin, dithiobutylamine (DTBA), cysteine, cysteine-thioglycolate, thioglycolic acid or hydroxyethyldisulphide (HED). Thereby proteins present in the sample are being further denatured by reducing disulphide linkages, thus overcoming some forms of tertiary protein folding, and breaking up quaternary protein structure (oligomeric subunits). This is known as reducing SDS-PAGE, and is the most commonly used technique in the art.

Silver staining was introduced by Kerenyi and Gallyas as a sensitive procedure to detect trace amounts of proteins in gels. The technique has been extended to the study of other biological macromolecules that have been separated in a variety of supports (Switzer, Merril et al. (1979), Anal Biochem. 98 (1), pages 231-237). Classical Coomassie Brilliant Blue staining can usually detect a 50 ng protein band, Silver staining increases the sensitivity typically about 50 times. Many variables can influence the colour intensity and every protein has its own staining characteristics.

Most protein separations are performed using a "discontinuous" (or "DISC") buffer system that significantly enhances the sharpness of the bands within the gel. During electrophoresis in a discontinuous gel system, an ion gradient is formed in the early stage of electrophoresis that causes all of the proteins to focus into a single sharp band. The formation of the ion gradient is achieved by choosing a pH value at which the ions of the buffer are only moderately charged compared to the SDS-coated proteins. These conditions provide an environment in which Kohlrausch reactions determine the molar conductivity. As a result, SDS-coated proteins are concentrated to several fold in a thin zone of the order of 19µm within a few minutes. At this stage all proteins migrate at the same migration speed by isotachophoresis. This occurs in a region of the gel that has larger pores so that the gel matrix does not retard the migration during the focusing or "stacking" event (Ornstein (1964), Ann. New York Acad. Sci 121 (2), pages 321-349; Davis (1964), Ann. New York Acad. Sci 121 (2), pages 404-427). Separation of the proteins by size is achieved in the lower, "resolving" region of the gel. The resolving gel typically has a much smaller pore size, which leads to a sieving effect that now determines the electrophoretic mobility of the proteins. At the same time, the separating part of the gel also has a pH value in which the buffer ions on average carry a greater charge, causing them to "outrun" the SDS-covered proteins and eliminate the ion gradient and thereby the stacking effect.

A very widespread discontinuous buffer system is the tris/glycine or "Laemmli" system that stacks at a pH of 6.8 and resolves at a pH of -8.3-9.0. In this system proteins are being denatured, i.e. ideally unfolded to a random coil, and encompassed by a micelle of an anionic surfactant such as SDS or LDS. Thereby differences in charge between proteins are at least essentially cancelled out. As a result proteins are at least essentially separated according to their size. The pH value for separation used in the Laemmli" system may allow unwanted disulphide bond formation between cysteine residues in proteins because the pKa of cysteine ranges from 8-9 and because reducing agent present in the loading buffer doesn't co-migrate with the proteins. The risk of the formation of disulphide bonds can be avoided by resolving the proteins at a pH value that is well below the pKa of cysteine and by including a reducing agent (e.g. sodium bisulfite) that moves into the gel ahead of the proteins to maintain a reducing environment. An illustrative example of a buffer compound with a pKa value below the pKa of cysteine is 2-[Bis(2-hydroxyethyl)amino]-2-(hydroxymethyl)-1,3-propanediol), abbreviated Bistris. This compound contains a tertiary amine and as a result has a pKa value of about 6.5 and can thus be used to maintain a buffer solution at a pH value of about 6.5. In addition, at a pH value lower than pH 8, micelles of an anionic surfactant, which may include a protein to be separated (supra), are shielded to a different extent from the electrical filed. As a result the time to resolution is reduced and the time available for separation is increased. An additional benefit of using a buffer with a lower pH value is that the acrylamide gel is more stable at lower pH values, so the gels can be stored for long periods of time before use (Schägger, von Jagow (1987), Anal Biochem. 166, 2, 368-379; Wiltfang, Arold et al. (1991), Electrophoresis 12, 5, 352-366).

As an electric field is applied, the anions (i.e. negatively charged sample molecules) migrate toward the positive electrode (anode), which is in a typical setup in the lower chamber. The leading ion is Cl⁻ (high mobility and high concentration), while glycinate is the trailing ion (low mobility and low concentration). SDS-protein particles do not migrate freely at the border between the Cl⁻ of the gel buffer and the Gly⁻ of the cathode buffer. Friedrich Kohlrausch found that Ohm's law also applies to dissolved electrolytes. Because of the voltage drop between the Cl⁻ and glycine-buffers, proteins are compressed (stacked) into micrometer thin layers. The boundary moves through a pore gradient and the protein stack gradually disperses due to a frictional resistance increase of the gel matrix. Stacking and unstacking occurs continuously in the gradient gel, for every protein at a different position. For a complete protein unstacking the polyacrylamide-gel concentration must exceed 16% T. The two-gel system of "Laemmli" is a simple gradient gel. The pH discontinuity of the buffers is of no significance for the separation quality, and a "stacking-gel" with a different pH is not needed.

When microorganisms such as bacteria are to be used as a whole cell preparation, the harvested cells may be processed using routine and known methods to inactivate the cells. A microorganism or microbe as used herein is a microscopic organism that includes a single cell (unicellular) or cell clusters. Microorganisms are very diverse and include bacteria, fungi, Archae, and protists; microscopic plants (green algae); and micro-animals such as plankton and the planarian. Some microbiologists also include viruses, whereas others consider these as nonliving. Most microorganisms are unicellular (single-celled), but this is not universal, since some multicellular organisms are microscopic, while some unicellular protists and bacteria are macroscopic and visible to the naked eye. The term "cultivation", as used herein, refers to the growth of microorganisms under controlled conditions, wherein the microorganisms are submersed, or on solid supports with the provided source of substrates needed for the growth of microorganisms. When a microorganism - which includes in the context of a method as described herein also viruses - or a eukaryotic cell is to be used to prepare polypeptides as defined herein, the microbe or the eukaryotic cell may be disrupted using chemical, physical, or mechanical methods routine and known to the art, including, for example, boiling, french press, sonication, digestion of peptidoglycan (for instance, by digestion with lysozyme), or homogenization. As used herein, "disruption" refers to the breaking up of the cell. Disruption of a microorganism can be measured by methods that are routine and known to the art, including, for instance, changes in optical density. Typically, a microbe is subjected to disruption until the percent transmittance is increased by 20% when a 1:100 dilution is measured. When physical or mechanical methods are used, the temperature during disruption is typically kept low, for example at about 4°C, to further minimize proteolytic degradation. When chemical methods are used, the temperature may be increased to optimize for the cell disruption. A combination of chemical, physical, and mechanical methods may also be used to solubilize the cell wall of the microbe.

An "aggregate" is a cluster of molecules which are mutually linked via non-covalent and/or covalent bonds, predominantly with hydrophobic as well as with other bonds (as, for example, disulfide bonds). These molecules are not biologically active. Insoluble aggregates of polypeptides may be isolated by methods that are routine and known to the art, for example, by centrifugation. Typically, centrifugation of insoluble polypeptides, such as membrane polypeptides, can be accomplished by centrifugal forces of 100,000 x g and more. Such centrifugal forces can be achieved by the use of ultracentrifuges.

The sample to be separated by electrophoresis contains a chaotropic agent. As indicated above, a chaotropic agent is typically used to improve solubility of certain proteins of comparable low polarity in an aqueous medium. The capability of a compound, in particular a salt, to precipitate proteins has been classified in the so called "Hofmeister series". In the following lines (A) and (B) salts that include an ion located toward the left are increasingly antichaotropic - also called kosmotropic - wherease salts that include an ion located toward the right are increasingly chaotropic (these are only partial listings of classified salts). Antichaotropic compounds induce protein aggregation and precipitation in aqueous solution. Ammonium sulfphate is one of the most common salts used for protein precipitation. Chaotropic salts, i.e. salts based on ions toward the right of each of the following lines (A) and (B), in contrast, reduce hydrophobic interactions as well as van der Waals forces and hydrogen bonds, thereby increasing the likelihood that a protein is kept in solution:
(A) SO₄²⁻ > CH₃COO⁻ > Cl⁻ > NO₃⁻ > Br⁻ > ClO₄⁻ > I⁻ > SCN⁻
(B) NH₄⁺ > K⁺ > Na⁺ > CS⁺ > Li⁺ > Mg²⁺ > Ca²⁺ > Ba²⁺ > Guanidinium⁺

It has previously been suggested that the higher the tendency of a cation is to be water-structure breaking, the higher is the stability of the cation's bond to dodecylsulfate and its exchange at the SDS micelle surface (Jakubowska, A, ChemPhysChem [2006] 7, 2312-2318). Chaotropic salts based on a chaotropic anion, that is salts toward the right of the above line (A), have recently been reported (Klaus, A, et al., Langmuir [2011] 27, 4403-4411) to enhance the transition from a clear to a turbid solution of the surfactant X-AES (C₁₂₋₁₄-PO₁₆-EO₂-SO₄Na). All chaotropic salts of the above line (A) were furthermore found to cause a transition from micellar solution to a solution with large macroscopic emulsion droplets. In view of these reports it appears plausible that the presence of chaotropic salts such as guanidinium salts affects a micellar solution of a conventional detergent such as SDS or LDS. Without being bound by theory, a method as disclosed herein is believed to circumvent the effects of such a combination of a conventional detergent and a chaotropic salt.

In some embodiments a chaotropic salt used as a chaotropic agent is a thiocyanate such as ammonium thiocyanate or calcium thiocyanate. In some embodiments the chaotropic salt is magnesium thiocyanate. The chaotropic salt is in some embodiments lithium thiocyanate. In some embodiments a chaotropic salt used as a chaotropic agent is a iodide such as lithium iodide. Two further illustrative examples of an iodide are calcium iodide and magnesium iodide. The chaotropic salt may also be a perchlorate such as sodium perchlorate. In some embodiments the chaotropic salt is a bromide such as sodium bromide or potassium bromide. In some embodiments the chaotropic salt is lithium bromide or calcium bromide. In some embodiments a chaotropic salt used as a chaotropic agent is a nitrate such as ammonium nitrate or potassium nitrate. A respective nitrate may also be sodium nitrate. The chaotropic salt is in some embodiments lithium nitrate. In some embodiments the chaotropic salt is calcium nitrate. In some embodiments a chaotropic salt used as a chaotropic agent is a guanidinium salt.

In a method described herein a buffer may be added to a sample. A large variety of different buffers is used in the art for preparing a sample for electrophoresis, and any of these sample buffers may be employed in a method as described herein. A respective buffer may for instance include one or more salts, a dye and/or a detergent in any desired concentration, as long as the concentration of the respective salt, dye and/or detergent allows this compound to remain in aqueous solution. A buffer as included in the sample buffer typically stabilizes the pH value to the desired value within the gel itself and in the electrophoresis buffer. The choice of buffer also affects the electrophoretic mobility of the buffer counterions and, thereby, the resolution of the gel. As evident to those skilled in the art, the buffer should also not modify or react with most proteins. Different buffers may be used as cathode and anode running buffers, respectively, depending on the application. Multiple pH values may be used within a single gel, for example in DISC (discontinuous) electrophoresis. Numerous buffer compounds are used in the art and may be used to perform a method described herein. Suitable buffers used in the electrophoresis as described herein include, but are not limited to, for example, solutions of a salt of phosphate, carbonate, succinate, citrate, acetate, formate, barbiturate, oxalate, lactate, phthalate, maleate, cacodylate, borate, triethanolamine, diethanolamine, ethylamine, triethylamine, glycine, glycylglycine, histidine, N-(2-acetamido)-2-amino-ethanesulfonate (also called (ACES), tris(hydroxymethyl)aminomethane (also called tris), bis-(2-hydroxyethyl)-imino-tris(hydroxymethyl)methane (also called Bistris), 3[N-tris-hydroxymethyl-methylamino]-2-hydroxypropanesulphonic acid (TAPSO), 1,4-piperazinediethanesulphonic acid (PIPES), imidazole, 2-(N-morpholino)ethanesulfonic acid (MES), 3-(N-Morpholino)propanesulfonic acid (MOPS), (2-[Tris(hydroxymethyl)-methylamino]-1-ethansulfonic acid (also called TES), N,N-bis[2-hydroxyethyl]-2-aminoethanesulphonic acid (BES), 2-cyclohexylamino-ethansulfonic acid (also called CHES) and N-(2-acetamido)-iminodiacetate (also called ADA), N-[Tris(hydroxymethyl)-methyl]-glycine (also called TRICINE), N,N-Bis(2-hydroxyethyl)-glycine (BICINE) and 4-(2-Hydroxyethyl)piperazine-1-ethanesulfonic acid (HEPES). In some embodiments a zwitterionic buffer such as for example 4-(2-hydroxyethyl)piperazine-1-propanesulfonic acid (EPPS), 3-(cyclohexylamino)-2-hydroxy-1-propanesulfonic acid, 2-amino-2methyl-1,3-propanediol (AMPD), 3-(cyclohexylamino)-1-propanesulfonic acid (CAPS), or N-Glycylglycine may be used (cf. international patent application WO 2011/134072). In some embodiment, the buffer in the electrophoresis sample buffer is tris. In some embodiments tris is used in a final concentration of about 0.01 M to about 0.3 M. The pH of the buffer may for example be selected in the range from about 6.5 to about 9.0. The pH may be adjusted using a suitable acid or base. In the art typically HCl is used for reducing the pH to a desired value. As an illustrative example, in one embodiment a final concentration of about 0.0625 M tris, pH 8.45 is used in the electrophoresis sample buffer.

To a sample used in a method described in this document, N-Lauroylsarcosine (2-[dodecanoyl(methyl)amino]acetic acid)" (C₁₅H₂₉NO₃, CAS number 97-78-9), or a salt of the latter is being added, the sodium salt of which is abbreviated as sodium N-Lauroylsarcosinate (SARCOSYL, N-Dodecanoyl-N-methylglycine sodium salt, CAS number 137-16-6). The sodium salt of N-Lauroylsarcosine is an anionic tenside and shows, in contrast to SDS, good solubility in solutions that contain a high concentration of a guanidinium salt, in particular guanidinium hydrochloride, and can, therefore, be used as a detergent, e.g., in a guanidinium-containing cell lysis buffer. In addition, N-Lauroylsarcosine is easy to dissolve in a sample buffer and in a running buffer and shows excellent overall compatibility with many electrophoretic buffer systems. In typical embodiments N-Lauroylsarcosine is being added to the sample together with further matter. In such embodiments N-Lauroylsarcosine may be added to the sample as a component of a sample buffer. In the context of sample preparation for electrophoresis the use of a sample buffer is standard practice in the art. A sample buffer may be added to the sample in a certain ratio and/or up to a total volume. Thus the sample buffer may also be used to adjust the volume and concentration of different samples to at least essentially the same value. Where a respective sample buffer is used, the sample buffer may include N-Lauroylsarcosine. In some embodiments the concentration of N-Lauroylsarcosine is selected in the range from about 0.2 to about 10 % (w/v), such as about 0.2 to about 5 %. In some embodiments the sample buffer contains about 0.5 % N-Lauroylsarcosine or more. In some embodiments the concentration of N-Lauroylsarcosine in the sample buffer is about 1% or more. As a further example, the sample buffer may contain about 2.5 % N-Lauroylsarcosin or more. In some embodiments the concentration of N-Lauroylsarcosine in the sample is about 3 % or higher. As an illustrative example, the sample buffer may have a pH of about 8.5 and include about 3 % (w/v) N-Lauroylsarcosine. In some embodiments a sample buffer used may have a concentration, again (w/v), of about 4 % N-Lauroylsarcosine or more, such as 4.5 % or more. The sample buffer may also include about 5 % (weight/volume; w/v) or more N-Lauroylsarcosine. In some embodiments N-Lauroylsarcosine is used in a concentration from about 1 % to about 4 % (w/v) in the sample buffer. As noted above, unless stated otherwise, all indicated concentrations are final concentrations, that is a concentration after addtition to the sample or of the sample.

To the sample containing the chaotropic agent, the guanidinium salt, and/or the potassium salt, sample buffer is added before the sample is applied to a gel such as a polyacrylamide gel or an agarose gel, or to a separation buffer for electrophoretic separation. The sample is typically mixed with the sample buffer and brought to an elevated temperature before being applied to the separation system, e.g. loaded onto the gel. Those familiar with electrophoresis will be aware of the fact that in some embodiments varying amounts of a stock solution of the sample buffer can be added to a sample before it is being applied to the separation system, for example in order to adjust the sample volume in gel pockets. As an illustrative example, a polyacrylamide gel may have pockets with a volume of 50 µl, into which the samples are being loaded. To a sample with a volume of 25 µl there may then be added 25 µl of a stock solution of the sample buffer. The stock solution may have 2-fold concentrations of the components defined herein, so that the final concentrations of the components of the sample buffer are attained upon addition of the sample. To a sample of 15 µl there may be added 10 µl of water, as well as 25 µl of a stock solution that contains 2-fold concentrations of the components defined herein. In some embodiments to a sample of 15 µl there may be added 35 µl of the respective 2-fold stock solution of sample buffer. Where desired the sample buffer may be diluted before being added to a sample of small volume such as 15 µl in the above example, to maintain a constant concentration of buffer components being added to the sample. For sake of convenience in some embodiments varying amounts of sample buffer may be added to the sample. In some embodiments a final concentration of about 0.05 % to about 6 % (w/v) of N-Lauroylsarcosine may be present in the sample before it is being exposed to electrophoretic analysis. In some embodiments a final concentration of about 0.08 % to about 5 % (w/v) of N-Lauroylsarcosine is present in the sample before electrophoretic analysis. As an illustrative example, about 3% (w/v) N-Lauroylsarcosine may be used as a final concentration in the gel electrophoresis sample buffer. In some embodiments a final concentration of about 0.08 % to about 5 % (w/v) of N-Lauroylsarcosine is included in a sample to be analyzed by electrophoresis.

As explained above, a reducing agent is added to the sample. The reducing agent may be included in the sample buffer, which is added to the sample. The reducing agent may be added to the sample independent from the addition of N-Lauroylsarcosine. In some embodiments the reducing agent is added together with N-Lauroylsarcosine. The chelating agent may be added independent from the addition of N-Lauroylsarcosine. The chelating agent may be added independent from the addition of the reducing agent. In some embodiments the chelating agent may be included in the sample buffer, which is added to the sample. For ease of reference throughout this document embodiments are referred to where the reducing agent and, if present, the chelating agent are included in the sample buffer.

As an illustrative example in this regard, an electrophoresis sample buffer used in a method described in this document may include, essentially consist of or consist of 0.0625M tris, pH 8.45, 3% (w/v) N-Lauroylsarcosine, 20% (v/v) glycerol, 100 mM DTT, 0,001% (w/v) BPB and 3mM EDTA. Indicated are the concentrations of the respective components of the sample buffer after the sample has been added thereto.

As indicated above, after the sample buffer has been added to the sample, the sample is in some embodiments briefly heated, for example to a temperature in the range from about 40 to about 100 °C, generally for a predetermined period of time. In some embodiments the sample is brought to a temperature in the range from about 65°C to about 75°C. The sample may be kept at the elevated temperature for 10 minutes or more, such as 15 minutes or more. The sample may be kept at the elevated temperature for about 1 to about 12 minutes. In some embodiments the sample is kept at the elevated temperature for about 5 to 10 minutes. In one illustrative embodiment the sample is heated to a temperature of about 70°C for ten minutes. Heating of the sample in the presence of a reducing agent such as DTT further denatures the proteins by reducing disulfide bridges, as explained in more detail elsewhere herein. The sample preparation may also include a procedure for an alkylation of amino groups of the sample proteins as known in the art, e.g. a blocking of thiols by treatment with iodoacetamide.

Subsequently the sample may be loaded to a separation device, for example to a polyacrylamide gel, an agarose gel or a flow chamber. The polyacrylamide gels as used herein may correspond to those described in the art, which may be used as commercially available or of which stock compositions- may be used as commercially available. In such an embodiment the gel may be prepared and cast according to the manufacturer's instructions.

Finally, the sample is separated in a gel or in a free-flow electrophoresis apparatus. In gel electrophoresis, the sample is separated in the gel in a gel electrophoresis running buffer that contains about 0.01 % (w/v) N-Lauroylsarcosine or more as a final concentration in order to analyze the protein sample, in the presence of a guanidinium salt, a potassium salt and/or a chaotropic agent. In some embodiments the running buffer contains about 0.03 % (w/v) N-Lauroylsarcosine or more. In some embodiments the running buffer contains N-Lauroylsarcosine in an amount from about 0.01 % to about 0.1 % (w/v). The running buffer may contain N-Lauroylsarcosine in an amount from about 0.01 % to about 0.1 % (w/v), such as from about 0.01 % to about 0.05 % (w/v). In some embodiments the running buffer contains N-Lauroylsarcosine in an amount from about 0.01 % to about 0.035 % (w/v).

As a buffer compound, e.g., tris-base, tris/Cl, tris/acetate, tris/borate, tris/glycine, tris/tricine, MOPS or tris/MOPS, MES, tris/MES, or HEPES, and also a zwitterionic buffer such as for example 4-(2-hydroxyethyl)piperazine-1-propanesulfonic acid (EPPS) or 3-(cyclohexylamino)-2-hydroxy-1-propanesulfonic acid, or N-Glycylglycine (supra, cf. also international patent application WO 2011/134072) can be used. In some embodiments the running buffer in addition to N-Lauroylsarcosine contains a chelating agent, such as EDTA, a buffer compound, e.g. tris, tris/glycine, tris/tricine, tris/acetate, tris/borate, MOPS or tris/MOPS, MES, HEPES or a zwitterionic buffer compound. The buffer may also include SDS and/or sodium-bisulfite. As indicated above, the electrophoresis may be carried out for example according to Bistris system, the tris/glycine system, the tris/tricine system or other PAGE systems of the art. In particular in embodiments where the bistris system is employed, EDTA may for instance be used a running buffer in a final concentration of about 0.5 mM to about 10 mM. A running buffer compound such as tris may in some embodiments be used in a final concentration of about 0.02 to about 0.2 M. The pH of the running buffer, when carrying out a Bistris system may in some embodiments be selected in the range from about 7.0 to 8.5, depending on the type of gel system used. MOPS as a running buffer compound may for example be used in a final concentration of about 0.3 to about 0.7 M. SDS may in some embodiments be present in a final concentration from about 0.05 to 0.2 %. Sodium bisulfite may be included in the running buffer in e.g. a final concentration from about 0.5 to about 3 mM. The running buffer in some embodiments includes, essentially consists of, or consists of 0.05M tris, pH 7.7, 0.05 M MOPS, 0.1% (w/v) SDS, 0.01% (w/v) N-Lauroylsarcosine, 1mM EDTA and 1mM sodium bisulfite (corresponding to the final concentrations of the respective components).

When carrying out electrophoresis according to the Bistris system, the gel buffer used in carrying out electrophoresis is typically Bistris. In some embodiments about 0.2 M to about 0.5 M of Bistris are used as final concentrations, both for the stacking and resolving gel. The pH is in typical embodiments selected from about 6.0 and about 7.5. The pH may in some embodiments be set within the range from about 6.5 and about 7.2. In some embodiments the gel buffer includes, essentially consists of, or consists of about 0.36 M Bistris, pH 6.7, as a final concentration.

In embodiments where electrophoresis is to be carried out according to the Laemmli method using the tris/glycine buffer system, gels may be prepared in a similar way as in the well known procedures in the art. The inventors have found that, when compared to conventional electrophoresis, using a gel buffer with an increased concentration of the buffer compound used - such as tris - generally allows a particularly good separation and resolution of proteins. Such a gel buffer may be used for both the stacking gel and for the separating gel. In some embodiments a gel buffer may contain from about 0.002 to about 0.05 (w/v) N-Lauroylsarcosine. A gel buffer may also contain about 0.5 to about 1.5 M tris. A gel buffer may furthermore contain SDS, for example in the range from about 0.05 to about 1 % (w/v). In one embodiment both the stacking and the separating gel may contain about 0.1 % (w/v) SDS, about 1.125 M tris, and have a pH value of about 8.8. If the tris/glycine buffer system is employed, furthermore the running buffer may contain about 25mM tris, about 190 to about 200 mM glycine, about 1.5 mM EDTA and about 0.1 % (w/v) SDS. The running buffer in this system may furthermore in some embodiments contain about 0.002 to about 0.06 (w/v) N-Lauroylsarcosine. In some embodiments a respective running buffer may contain about 0.01 % to about 0.033 % (w/v) N-Lauroylsarcosine. The pH of the running buffer is typically not being adjusted. It may for example have a value of about 7.6 or more. In one embodiment the pH of the gel buffer may be about 8.3.

In embodiments where electrophoresis is carried out according to the tris/tricine system (according to Schaegger) the gel buffer may be used according to the well known protocol used in the art. Both the stacking and the separating gel may include about 1.0 M tris, and about 0.1 % (v/v) SDS. To adjust the pH, about 0.33 M HCl may be included. The pH of the gel buffer may for example be set to about 8.45. When using the tris/tricine system, the anode running buffer may contain about 100 mM tris and the pH may be adjusted by HCl to about 8.9. In one embodiment the anode running buffer may contain about 100 mM tris, about 1.5 mM EDTA, and about 0.01 % to about 0.033 % (w/v) N-Lauroylsarcosine. The cathode running buffer may contain 100 mM tris, about 100 mM tricine, about 1.5 mM EDTA, about 0.1 % (w/v) SDS and about 0.01 % to about 0.033 % (w/v) N-Lauroylsarcosine.

A protein or polypeptide analyzed in a method disclosed herein may be any protein. In some embodiments a respective protein originated from a cell. The respective protein may be a heterologous protein, i.e. a protein that is foreign to the organism in which expression is performed. In some embodiments such a protein may be a fusion protein, i.e. a protein fused to another peptide or protein, including an affinity purification tag, such as, e.g., 6xHis -tag, glutathione-S-transferase (GST), small ubiquitin-like modifier (SUMO), thioredoxin or maltose binding protein (MBP) and the like. Affinity purification tags can be fused to any recombinant protein of interest, allowing fast and easy purification using the affinity properties of the tag. Certain tags are used because they encode an epitope that can be purified or detected by a specific antibody or because they enable simplified purification of a desired protein. Researchers often need to purify a single protein for further study. One method for isolating a specific protein is the use of affinity tags. Many different affinity tags have been developed to simplify protein purification. Fusion tags are polypeptides, small proteins or enzymes added to the N- or C-terminus of a recombinant protein. The biochemical features of different tags influence the stability, solubility and expression of proteins to which they are attached. Using expression vectors described in the art that include a fusion tag facilitates recombinant protein purification.

The most commonly used tag to purify and detect recombinant expressed proteins is the polyhistidine tag, e.g the 6xHis tag. Protein purification using polyhistidine tags relies on the affinity of histidine residues for immobilized metal ions such as nickel, zinc, or copper ions, or others which allows selective protein purification. This affinity interaction is believed to be a result of coordination of a nitrogen atom on the imidazole moiety of polyhistidine with a vacant coordination site on the metal. The metal ion is immobilized to a support through complex formation with a chelate that is covalently attached to the support. Polyhistidine tags offer several advantages for protein purification, as appreciated by those skilled in the art. The small size of the polyhistidine tag renders it less immunogenic than other larger tags. Therefore, the tag usually does not need to be removed for downstream applications following purification. A large number of commercial expression vectors that contain polyhistidine are available. The polyhistidine tag may be placed on either the N- or C-terminus of the protein of interest. Further, the interaction of the polyhistidine tag with the metal does not depend on the tertiary structure of the tag, making it possible to purify otherwise insoluble proteins using denaturing conditions.

The use of the affinity tag glutathione-S-transferase (GST) is based on the strong affinity of GST for immobilized glutathione-covered matrices. Glutathione-S-transferases are a family of multifunctional cytosolic proteins that are present in eukaryotic organisms. GST isoforms are not normally found in bacteria; thus endogenous bacterial proteins do not compete with the GST-fusion proteins for binding to purification resin. The 26kDa GST affinity tag enhances the solubility of many eukaryotic proteins expressed in bacteria.

The methods described herein are particularly useful for the separation and analysis of such heterologous or fusion proteins.

In addition, a method as described herein provides an efficient technique for the separation and analysis of proteins embedded or included in inclusion bodies (supra).

E. coli have been most widely used for the production of recombinant proteins that do not require post-translational modification such as glycosylation for bioactivity. However, high-level expression of recombinant proteins in E. coli often results in accumulating them as insoluble aggregates in vivo as inclusion bodies. Inclusion body proteins are devoid of biological activity and need elaborate solubilization, refolding and purification procedures to recover functionally active product. In general, inclusion bodies are solubilized by the use of a high concentration of chaotropic agent such as urea or guanidine hydrochloride, along with a reducing agent such as β-mercaptoethanol. Solubilized proteins are then refolded by slow removal of the chaotropic agent in the presence of oxidizing agent. Protein solubilization from the inclusion body using high concentrations of chaotropic reagents results in the loss of secondary structure leading to the random coil formation of the protein structure and exposure of the hydrophobic surface. Loss of secondary structure during solubilization and the interaction among the denatured protein molecules during refolding resulting in their aggregation are considered to be the main reasons for the poor recovery of bioactive proteins from the inclusion bodies. Many times, the overall yield of bioactive protein from inclusion bodies is around 15 - 25% of the total protein and accounts for the major cost in production of recombinant protein from E. coli.

Thus, a major bioprocess engineering challenge has been to convert this inactive and insoluble protein more efficiently into soluble and correctly folded product. The recovery of bioactive therapeutic protein from inclusion bodies described in the art involves four steps: isolation of inclusion bodies from E. coli cells; solubilization of protein aggregates; and refolding and purification of the solubilized protein.

Among these steps, solubilization and refolding are the most crucial steps for high recovery of bioactive protein. Inclusion bodies are generally separated from the cell debris using low-speed centrifugation after cell lysis as they are denser than many of the cellular components. Semipure protein aggregates along with contaminants are then solubilized using high concentrations (6 - 8M) of a chaotropic reagent, such as urea, guanidine hydrochloride and a detergent such as SDS, N-acetyl trimethyl ammonium chloride and N-Lauroylsarcosine. This helps to maintain cysteine residues in a reduced state and thus prevents non-native intra-or inter-disulfide bond formation in highly concentrated protein solutions at alkaline pH. Chelating agents like EDTA are frequently used in the solubilization buffer to prevent metal-catalyzed air oxidation of cysteines. Solubilized proteins are then refolded into their native state during the removal of the chaotropic reagent. Refolding followed by purification is generally advantageous as some of the high molecular weight aggregates along with contaminants can be co-purified in a single step.

Protein aggregation is one of the major problems associated with the process of refolding inclusion body proteins. Aggregation results from intermolecular interaction that competes with intramolecular reaction. Aggregates mostly form by non-native hydrophobic interactions between the folding intermediates in which the hydrophobic patches are exposed. Solubilization of protein aggregates in a high concentration of chaotropic agents generates random coil structure of the protein where such hydrophobic amino acid stretches are exposed. This enhances the propensity of aggregation during refolding. One of the ways to reduce protein aggregation is to have a refolding process in which the intermediates are beyond the aggregated prone structure, i.e., where the hydrophobic patches are not fully exposed. This can be achieved by mild solubilization of inclusion body proteins without generating the random coil configuration of the configuration of the protein. Prevention of hydrophobic interactions during the initial stages of refolding is thus crucial for lower protein aggregation and improved renaturation of inclusion body protein.

Proteins are usually engineered to be overexpressed in Escherichia coli as fusion proteins, commonly with glutathione S-transferase (GST), small ubiquitin-like modifier (SUMO), thioredoxin and maltose binding protein (MBP). These fusion systems promise increased solubility of target proteins, except for the 6xHis tag, with single-step purification efficiency. However, many recombinant proteins, especially those of eukaryotic origin, aggregate or become packaged into inclusion bodies. Refolding recombinant proteins from inclusion bodies can be challenging and yields of correctly folded proteins can be low.

A method as disclosed herein is particularly advantageous in that it allows protein samples that are brought into a state of solution by means of a chaotropic agent, such as guanidinium hydrochloride, to be directly used for gel electrophoresis such as agarose gel electrophoresis or PAGE, e.g. SDS-PAGE, or for free-flow electrophoresis. Accordingly, the removal of the chaotropic agent, for example, by dialyzing against an appropriate buffer by methods known in the art (for instance, diafiltration, precipitation, hydrophobic chromatography, ion-exchange chromatography, or affinity chromatography, or ultrafiltration and washing the polypeptides, for instance, in alcohol, by diafiltration) which was thus far necessary in the methods in the art prior to SDS-PAGE can be avoided. The methods disclosed in this document are particularly suitable for the analysis of recombinant, tagged proteins, especially those of eukaryotic origin, packaged into inclusion bodies, in light of the problems set forth above.

A method described in this specification can also be used for two-dimensional gel electrophoresis. Two-dimensional gel electrophoresis or briefly 2-D electrophoresis, is a form of gel electrophoresis commonly used to analyze proteins. In this method, mixtures of proteins are separated by two properties in two dimensions on 2-D gels. 2-D electrophoersis begins with 1-D electrophoresis but then separates the molecules by a second property, in a direction 90 degrees from the first. In 1-D electrophoresis, proteins (or other molecules such as DNA) are separated in one dimension, so that all the proteins/molecules will lie along a lane but that the molecules are spread out across a 2-D gel. Because it is unlikely that two molecules will be similar in two distinct properties, molecules are more effectively separated in 2-D electrophoresis than in 1-D electrophoresis. The two dimensions that proteins are separated into using this technique can be, for instance, isoelectric point, protein complex mass in the native state, and protein mass.

A technique used in the art to separate proteins by their isoelectric point is isoelectric focusing (IEF). When carried out as gel electrophoresis, in this technique a pH gradient is applied to a gel and an electric potential is applied across the gel, making one end more positive than the other. At all pH values other than their isoelectric point, proteins will be charged. If they are positively charged, they will be pulled towards the more negative end of the gel and if they are negatively charged they will be pulled to the more positive end of the gel. The proteins applied in the first dimension will move along the gel and will accumulate at their isoelectric point; that is, the point at which the overall charge on the protein is 0 (a neutral charge).

For analysing the function of a protein in a biological cell, knowledge of its interacting partners is essential. Most often proteins act together in complexes to be fully functional/biologically active. The analysis of this sub organelle organization of the cell requires techniques conserving the native state of the protein complexes. In native polyacrylamide gel electrophoresis (native PAGE), proteins remain in their native state and are separated in the electric field following their mass and the mass of their complexes, respectively. To obtain a separation by size and not by net charge, as in IEF, an additional charge is transferred to the proteins by the use of Coomassie Brilliant Blue or lithium dodecyl sulfate. After completion of the first dimension the complexes are destroyed by applying the denaturing SDS-PAGE in the second dimension, where the proteins of which the complexes are composed of are separated by their mass.

Before separating the proteins by mass, they are generally treated with SDS, along with other reagents, as described above (SDS-PAGE in 1-D). This denatures the proteins and binds a number of SDS molecules roughly proportional to the protein's length, as set forth above. Because a protein's length (when unfolded) is roughly proportional to its mass, this is equivalent to saying that it attaches a number of SDS molecules roughly proportional to the protein's mass. Since the SDS molecules are negatively charged, the result of this is that all of the proteins will have approximately the same mass-to-charge ratio as each other. In addition, proteins will not migrate when they have no charge (a result of the isoelectric focusing step), therefore the coating of the protein in SDS (negatively charged) allows migration of the proteins in the second dimension. Separation of denatured proteins in the first dimension of a 2-D electrophoresis has been performed e.g. after loading of the protein samples with different anionic or cationic detergents or different gel systems, thereby circumventing the problematic issue that a significant fraction of cellular proteins is not soluble in the absents of denaturing conditions. In the second dimension, an electric potential is again applied, but at a 90 degree angle from the first field. The proteins will be attracted to the more positive side of the gel (because SDS is negatively charged) proportionally to their mass-to-charge ratio. As previously explained, this ratio will be nearly the same for all proteins. The proteins' progress will be slowed by frictional forces. The gel therefore can be taken to act as a molecular sieve when the current is applied, separating the proteins on the basis of their molecular weight with larger proteins being retained higher in the gel and smaller proteins being able to pass through the sieve and reach lower regions of the gel.

The result of this is a gel with proteins spread out on its surface. These proteins can then be detected by a variety of means, such as silver staining or Coomassie Brilliant Blue staining. In the former case, a silver colloid is applied to the gel. The silver binds to cysteine groups within the protein. The silver is darkened by exposure to ultra-violet light. The amount of silver can be related to the darkness, and therefore the amount of protein at a given location on the gel. This measurement can only give approximate amounts, but is adequate for most purposes.

In quantitative proteomics, 2-D gel analysis software can be used to analyze bio-markers by quantifying individual proteins, and showing the separation between one or more protein "spots" on a scanned image of a 2-D gel. Additionally, such software matches spots between gels of similar samples to show, for example, proteomic differences between early and advanced stages of an illness. Software packages include, for example, BioNumerics 2D, Delta2D, ImageMaster, Melanie, PDQuest, Progenesis and REDFIN - among others. 2-D gel analysis software typically serves four purposes: analyze bio-markers by quantifying individual proteins; showing the separation between one or more protein "spots" on a scanned image of a 2-D gel; match spots between gels of similar samples to show, for example, proteomic differences between early and advanced stages of an illness; and in depth analysis.

In addition, the methods described in the specification have advantageously been established for use with various established and known gel/buffer systems, both in the presence and absence of a conventional detergent, as also demonstrated in multiple examples. Some of them are exemplified in the following.

For instance, the NuPAGE® Pre-Cast Gel System is a polyacrylamide gel system for high performance gel electrophoresis. It consists of NuPAGE® Bistris Pre-Cast Gels (for small to midsize molecular weight proteins), NuPAGE® tris/acetate Gels (for larger proteins) and specially optimized buffers. The unique formulation and low operating pH during electrophoresis offer significant advantages over other gel systems. While there are subtle differences between the chemistries of Bistris and tris/acetate gels, both provide a much lower pH environment than traditional SDS-PAGE systems. The advantages of lower pH include sharper band resolution, longer shelf-life, and higher accuracy of results. For many years, the Laemmli system has been the standard method used to perform SDS-PAGE. Laemmli-type gels (upon which the NOVEX® tris-glycine pre-cast gel chemistry is based) are useful for a broad range of protein separations. The tris/tricine gel system was later developed to extend the separation range to small peptides. As appreciated by those skilled in the art, the Laemmli system has certainly proved its worth, but it is subject to some potential problems.

Criterion™ XT gels are ideal for vertical midi gel electrophoresis and are designed to work with optimized sample and running buffers without the need for antioxidant addition. Like traditional Laemmli systems, Criterion™ XT gels use discontinuous buffer ion fronts that form moving boundaries to stack and then separate proteins.

Features and benefits of the Criterion™ XT gels described in the art are:
- Long shelf life (12 month).
- Formulated using a Bistris-HCl buffer system (pH 6.4) to separate proteins by molecular weight.
- Select from two running buffers to expand the separation capability of a single Bistris gel type.
- Near-neutral pH significantly delays acrylamide hydrolysis as compared to traditional Laemmli systems.
- Run 2-D gels in less than a day.

Criterion™ XT gels can be used, e.g., for polyacrylamide gel electrophoresis (PAGE or SDS-PAGE), 2-D gel electrophoresis, screening of new samples and for evaluating sample preparation conditions.

In the neutral Bistris-PAGE (Aces anode-buffer), a discontinuous SDS-PAGE system has been developed that operates under neutral conditions. [Bis(2-hydroxyethyl)imino]tris-(hydroxymethyl)methane (Bistris) buffers are employed with pH 6.8 and 6.3 for the separating and stacking gels, respectively, while the anode buffer N-2-acetamido-2-hydroxyethanesulfonic acid (Aces) and the Bistris cathode buffer both had a pH of 6.8. These conditions resulted in a relative trailing ion mobility of 0.349 and 0.137 in the resolving and stacking gel, respectively, under room temperature conditions. Peptides and proteins were resolved in the 3-100 kDa range with a 10% acrylamide resolving gel (Strating, Clarke (2001), Analytical Biochemistry 291, pages 149-154).

The neutral Bistris-PAGE using tris/MOPS or tris/MES running buffer has been described in Graham et al. (Proteomics [2005] 5, 2309-2314).

Further gel/buffer systems which can be used in combination with a method as described in this document are set forth elsewhere herein and in the following examples.

Provided is further an electrophoresis sample buffer, which may be a gel loading buffer, the composition of which is indicated elsewhere herein. This sample buffer is particularly useful for adjusting a chaotropic agent-containing-protein sample to denaturing electrophoresis, e.g. denaturing polyacrylamide gel electrophoresis (PAGE), including SDS-PAGE, where the sample is to be applied to an electrophoresis gel without any further processing. An electrophoresis sample buffer, including a PAGE sample buffer used in a method described herein may in one embodiment include, essentially consist of, or consist of about 0.0625M tris, pH 8.45, about 3% (w/v) N-Lauroylsarcosine, about 20% (v/v) glycerol, about 100 mM DTT, about 0.001% BPB and about 3 mM EDTA (again, final concentrations).

After the addition of the sample buffer to a protein sample containing a chaotropic agent such as guanidinium hydrochloride, the sample can be directly loaded to polyacrylamide gels under reducing and denaturing conditions. Specifically, the sample buffer encompasses N-lauroylsarcosine as an anionic agent which can be taken to substitute the commonly applied SDS. In addition, the sample buffer may contain DTT as a reducing agent and EDTA as a chelating agent. The pH in the sample buffer can be taken to be relatively high. If at all, the sample may only be mildly heated, i.e. brought to a temperature that is about e.g. 10 °C or 20 °C above room temperature, before loading. An only slightly elevated temperature may be desired in some embodiments in order to avoid oxidation and other changes of the proteins, such as inter-polypeptide aggregates.

Provided is also to an electrophoresis running buffer, which may also be used as a PAGE running buffer, including a SDS-PAGE running buffer. This running buffer basically corresponds to the running buffers used for polyacrylamide gel electrophoresis of the various gel/buffer systems established in the art. Nevertheless, for protein samples containing a chaotropic agent, a potassium salt, and/or a guanidinium salt, such as guanidinium hydrochloride, a modification has been found to be advantageous by the present inventors. This is the addition of N-lauroylsarcosine and a chelating agent such as EDTA to the running buffer. In a SDS-PAGE or LDS-PAGE running buffer, N-lauroylsarcosine is typically used in addition to an anionic detergent such as SDS, LDS or ε-aminocaproic acid. It is furthermore often advantageous in a method described in this specification where a protein sample containing a chaotropic agent, a potassium salt, and/or a guanidinium salt is to be analyzed, to extend the time chosen for allowing the sample to be separated electrophoretically, when compared to common SDS-PAGE used in the art. It may be advantageous to use a relatively long running time, when compared to standard gel electrophoresis used in the art. An extended time for separation is adjusted by applying a moderate voltage or a moderate current at the beginning of the electrophoretic process. The ions present in the guanidinium salt slow down the initial migration of the proteins into the gel matrix in the early sequence of electrophoresis. In embodiments where this effect becomes apparent, it is generally sufficient to start electrophoresis at a reduced current and/or voltage and subsequently rely on standard protocols used in the art. Once the proteins included in the sample have entered the gel, electrophoresis can thus generally be run with a commonly used voltage/current. Especially when high concentrations of guanidinium hydrochloride are being used, such as e.g. 6M guanidinium hydrochloride and if the loading volume of gel slots is almost entirely used, it may be advantageous to initiate electrophoresis at voltage/current settings that correspond to only a fractional amount of the settings commonly used. If starting with standard voltage/current, increased precipitates may form during the initial run in procedure, i.e. while proteins and peptides enter the polyacrylamide gel. Gel slots are for example almost entirely used, if 15 µl sample are applied to a gel slot that can maximally encompass about 20 µl. In the worst case scenario, the protein samples in the gel slots can be disturbed or may partially displaced. This effect can usually also be avoided by counteracting the SDS that is present in the running buffer by an addition of low amounts of N-Lauroylsarcosine to the running buffer described herein, so that the proteins included in the sample can run in slowly but properly.

In embodiments where a Bistris based system is to be employed for sample analysis, the running buffer may include, essentially consist of, or consist of about 0.05 M tris, about 0.05 M MOPS, about 0.1% (w/v) SDS, about 0.01 % to about 0.033 % (w/v) N-Lauroylsarcosine, about 1 mM EDTA and about 1 mM sodium bisulfite. These values correspond to the final concentrations.

In embodiments where electrophoresis is to be carried out using a Bistris based system, the gel buffer may include, at least essentially include or consist of about 0.357M Bistris, , as a final concentration. In such a system the pH value is known not to be critical, as long as extreme pH values, such as below 5.0 or above 11.0, are avoided. As an illustrative example, the pH value may be about 6.7.

In embodiments where a tris/glycine based system is to be employed, the running buffer may include, essentially consist of, or consist of about 25mM tris, about 190 to about 200 mM glycine, about 1.5 mM EDTA and about 0.1 % (w/v) SDS. The running buffer in this system furthermore contains about 0.01 % to about 0.033 % (w/v) N-Lauroylsarcosine. These values correspond to the final concentrations.

If electrophoresis is to be carried out using a tris/glycine based system, the gel buffer may include, at least essentially include or consist of about 0.1 % (w/v) SDS, about 1.125 M tris, and have a pH value of about 8.8.

In embodiments where a tris/tricine based system is to be employed, the anode running buffer may include, essentially consist of, or consist of about 100 mM tris as an aqueous solution. The anode running buffer may further include about 1.5 mM EDTA, and about 0.01 % to about 0.033 % (w/v) N-Lauroylsarcosine. The cathode running buffer may in some embodiments include, essentially consist of, or consist of about 100 mM tris, about 100 mM tricine, about 1.5 mM EDTA, about 0.1 % (w/v) SDS and about 0.01 % to about 0.033 % (w/v) N-Lauroylsarcosine.

Where electrophoresis is to be carried out using a tris/tricine based system, the gel buffer may include, essentially consist of, or consist of about 1.0 M tris, and about 0.1 % (w/v) SDS. The pH of the gel buffer may for example be set to about 8.45. As an illustrative example, about 0.33 M HCl may be included to adjust the pH.

Advantageously, the running properties of the gel electrophoresis using the buffers disclosed herein for chaotropic agent (e.g. guanidinium salt) containing samples are comparable to those of conventional electrophoresis methods. For example, the different polyacrylamide gel systems with various concentrations of acrylamide maintain the characteristic separation properties, e.g. the corresponding molecular-mass dependent migration distances of the proteins are typically well comparable to the standard PAGE systems used in the art and allow for a comparable illustration and a high resolution of single protein bands.

Provided is also an electrophoresis buffer system for the analysis of a protein sample, which contains a chaotropic agent, a guanidinium salt, and/or a potassium salt, and which includes one or more, such as two or all three of the buffers described in this document selected from the sample buffer, running buffer and gel buffer described above. The electrophoresis buffer system may in some embodiments be used as a denaturing electrophoresis system, e.g., as a denaturing polyacrylamide gel electrophoresis (PAGE) buffer system.

Provided is also a kit for gel electrophoresis analysis of a protein sample in the presence of a chaotropic denaturing agent (i.e. the protein sample contains one or more chaotropic agents), that includes at least one, two or all three of a running buffer, a sample buffer, and a gel buffer. A kit may also include a running buffer or a concentrate that can be diluted for obtaining a running buffer. A kit may also include a sample buffer or a concentrate that can be diluted for obtaining a sample buffer. In some embodiments a kit includes a gel buffer or a concentrate that can be diluted for obtaining a gel buffer. The kit can contain further components required for gel electrophoresis analysis, such as a pre-cast gel or a manual. In some embodiments a kit includes all reagents required to prepare the sample and to carry out electrophoresis. In some embodiments the kit includes a separation gel buffer, a stacking gel buffer, an acrylamide solution and a reservoir buffer that are adapted to carry out electrophoresis according to Laemmli using a tris/glycine based system. In some embodiments the kit includes a separation gel buffer, a stacking gel buffer, an acrylamide solution and a reservoir buffer (also called running buffer) that are adapted to carry out electrophoresis according to Laemmli using a tris/glycine based system. In some embodiments the kit includes a precast gel and a reservoir buffer (also called running buffer), which are adapted to carry out electrophoresis according to Laemmli using a tris/glycine based system. In some embodiments the kit includes a separation gel buffer, a stacking gel buffer, an acrylamide solution and a reservoir buffer (also called running buffer) that are adapted to carry out electrophoresis using a Bistris based system. In some embodiments the kit includes a precast gel and a reservoir buffer, which are adapted to carry out electrophoresis using a Bistris based system. In some embodiments the kit includes a separation gel buffer, a stacking gel buffer, an acrylamide solution, a cathode buffer and an anode buffer that are adapted to carry out electrophoresis using a tris/tricine based system according to Schaegger. In some embodiments the kit includes a precast gel, a cathode buffer and an anode buffer, which are adapted to carry out electrophoresis using a tris/tricine based system according to Schaegger.

After electrophoretic separation according to the methods, the gels, fractions and/or proteins can be used for all subsequent applications in known kind. For example, a gel can be stained by Coomassie Brilliant Blue stain, including the Colloidal Coomassie Blue stain, or by silver staining. Furthermore, detection of proteins by Western blot analysis and immunoblotting methods can be carried out when using the buffers described herein, without any detectable restraints. Furthermore, any other subsequent application that is generally performed after denaturing gel electrophoresis as used in the art can also be performed subsequent to an electrophoretic separation under conditions as described in this specification.

A method described in this specification allows the direct analysis of samples containing a chaotropic agent, a potassium salt, and/or a guanidinium salt, such as guanidinium hydrochloride by electrophoresis such as gel electrophoresis, without any further purification process. Thereby, time, material and labour can be saved which are otherwise necessary for purification of protein samples prior to established electrophoresis. The direct application of chaotropic agent-containing protein samples avoids qualitative and quantitative losses since only this way the proteins can be used and analyzed completely and without loss of material.

Provided is also the use of the buffers disclosed herein for the analysis of a protein sample that includes a chaotropic agent, a potassium salt, and/or a guanidinium salt by electrophoresis, such as denaturing SDS-polyacrylamide gel electrophoresis (PAGE).

All references cited in this specification are herewith incorporated by reference with respect to their entire disclosure content and the disclosure content specifically mentioned in this specification. In case of conflict, the present specification, including definitions, will control.

The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the appending claims. In addition, where features or aspects of the invention are described in terms of Markush groups, those skilled in the art will recognize that the invention is also thereby described in terms of any individual member or subgroup of members of the Markush group.

The following examples are only intended to illustrate the present invention. They shall not limit the scope of the invention in any way.

### EXAMPLES

### I. Sample preparation:

Recombinant protein extracts from *E. coli* lysates were prepared by applying standard methods for preparation of 6xHis-tagged fusion proteins. In brief, transformation of competent *E. coli* strains (Stratagene) was transformed with expression vectors pET15b (Novagen) or pQE32 (Qiagen, Hilden, Germany) containing truncated coding sequences of human p120 catenin (p120) or MRP14 (S100A9), respectively. Expressions from these vectors generate fusion proteins in which six consecutive histidine residues and a short linker sequence are fused to the amino terminus of the coding sequence, leading to recombinant fusion protein constructs (6xHis-tagged proteins) of 19 kDa and 100 kDa, respectively. Grown bacteria were induced with Isopropyl β-D-1-thiogalactopyranoside to express the vector-encoded sequences for 6 hours. Subsequently, cells were harvested by centrifugation and were frozen at -40°C. Cell lysis and protein solubilization was performed as suggested for the purification of 6xHis-tagged proteins with subsequent use of the Ni-NTA technology (Qiagen, Hilden, Germany) in guanidinium hydrochloride-lysis buffer (6M guanidinium hydrochloride, 10mM tris-HCl, 100mM NaH₂PO₄ pH 8.0) supported by sonication. Cell debris was removed by centrifugation (10,0000 xg). Protein concentration of the resulting soluble protein extracts were determined using a Coomassie Brilliant Blue G-250 based Protein Assay (BioRad Laboratories, München, Germany) and adjusted to indicated concentrations by dilution with the guanidinium hydrochloride-lysis buffer. As a result, a variety of protein extract samples each containing one of two different 6x-tagged proteins with divergent molecular mass and different total protein concentrations were available for the tests, all samples containing a total concentration of at least 6 M guanidinium hydrochloride. In cases of reduced concentrations 6M guanidinium samples were diluted with phosphate buffered saline.

### II. Acrylamide gel composition and casting:

PAGE was performed using small format gels, we used hand cast polyacrylamide gels for the Mini-Protean™ cell system, with 10-well comb and 1 mm spacer thickness, or precast Criterion™ XT Bistris gels of 1mm thickness using a 12+2 well comb (Bio-Rad Laboratories, Munich, Germany). Hand cast polyacrylamide gels were prepared using a ready-to-use acrylamide:bisarcrylamide mixture 37.5:1 (Rotiphorese Gel 30, Carl Roth, Karlsruhe, Germany) with the respective gel buffer (detailed gel buffer compositions are listed in Fig. 1), the polymerization was initiated by TEMED and ammonium persulfate. In the examples shown we performed generally three different gel-buffer systems, which are well known and described as Laemmli method (tris/glycine buffer system) or Schaegger method (tris/tricine buffer system) and the neutral Bistris-gel method, as known in the art or as provided commercially as precast gels. In detail, the tris/glycine PAGE was performed as described (Laemmli, 1970), or by using the modified tris/glycine system as described herein using considerable higher concentrations of tris-HCl (1.125 M) in the gel buffer (Laemmli-H). The protocol for tris/tricine based SDS-PAGE and gel casting was performed as reviewed (Schaegger and Jagow, 1987; Schaegger, 2006). Bistris gels comprising a neutral pH Bistris gel buffer were either purchased (Bio-Rad Laboratories, München, Germany) or hand-cast essentially as reported previously by others (Starting and Clarke, 2001; Graham et al., 2005). The detailed compositions of the different electrophoresis gel buffers and running buffers as used in the examples are reported in the tables of Fig. 2 and Fig. 3 described herein.

### III. Loading of samples and gel electrophoresis:

Before loading to polyacrylamide gels the protein samples were mixed with an equal volume of 2x concentrated sample buffer, mixed and heated for 3 minutes at 95°C or for 10 minutes at 70°C, as indicated. The protein samples were either applied with SDS-sample buffer of the well known Laemmli method, or with N-Lauroylsarcosine-containing sample buffer as disclosed herein. Sample buffer compositions are described in Fig. 1.

Lyophilized protein mixtures of molecular mass standards SDS6H2 for high molecular mass (HM) and SDS7 for low molecular mass (LM) (Sigma-Aldrich, Taufkirchen, Germany) or a ready to use standard for broad range molecular mass (BM) (PageRuler™, Thermo Scientific) were prepared according to the suppliers instruction and completed with an equal volume of the respective sample buffer as indicated. For a direct analysis of guanidinium hydrochloride samples, the molecular mass standards were supplemented with concentrated guanidinium hydrochloride-lysis buffer to achieve a guanidinium concentration identical to the applied protein samples. In the illustrations depicted in the appended figures, samples of a variety of protein concentrations (µg of total protein loaded as indicated) were applied on each gel, using 8%, 10%, 12%, 13%, or 16% acrylamide resolving gel with a 3.5% or 4% stacking gel, or using 4-to-12% gradient polyacrylamide gels. 15 µl or 20 µl of samples were loaded under cathode running buffer into the gel slots of hand-cast gels or Criterion™ XT precast gels (Bio-Rad Laboratories), respectively. Electrophoresis was performed using a running buffer as indicated in Fig. 2 and subjected to constant electrical current conditions (15-50 mA) with an initially reduced current until the samples had entered the gel. The protein bands were visualized by staining the gel with a colloidal preparation of Coomassie Brilliant Blue G250 dye (Carl Roth, Karlsruhe, Germany).

For immunodetection protein extracts were separated by PAGE as described herein and electro-transferred to nitrocellulose (Amersham-GE Healthcare, Freiburg, Germany) using a Trans-Blot semi-dry transfer cell (Bio-Rad Laboratories) as recommended by the supplier. Immunochemical detection of recombinant 6xHis-tagged proteins on the nitrocellulose membrane was performed using antibodies specific for S100A9 and p120catenin, and specific HRP-conjugated secondary antibodies essentially as previously described (Schnaeker et al., 2004, Cancer Res. 64, page 8924-31). Bound secondary antibodies were visualized on exposed x-ray film using the enhanced chemiluminescence detection reagents and detecting system (ECL, Amersham-GE Healthcare, Freiburg, Germany).

### Example 1

Fig. 4 shows the results of a polyacrylamide gel electrophoresis for direct analysis of proteins samples containing guanidinium hydrochloride as the chaotropic agent, as described herein. Protein samples from two different bacterial extracts containing 6xHis-tagged recombinant fusion proteins solubilized in 6M guanidinium hydrochloride were separated using the Schaegger system (tris/tricine-PAGE) with 10% polyacrylamide gels under denaturing conditions. For the sample buffer used refer to Fig. 1. In order to demonstrate the performance of the invented PAGE system the samples containing 6 M guanidinium hydrochloride were directly analyzed under conditions as described herein. A tris/tricine electrophoresis running buffer was used as shown in Fig. 2, containing 0.01% N-Lauroylsarcosine in the cathode buffer. To compare the quality of protein separation and resolution, an identical set of samples were depleted from guanidinium hydrochloride by precipitation using the TCA method. For these samples electrophoresis was performed under established buffer conditions (Schaegger method). Initial samples containing defined concentrations of the original bacterial extracts (µg of total protein as indicated above the lanes). HM and LM indicate standard protein mixtures of high and low molecular mass, respectively, which were used to define the protein mobility during electrophoresis. Calibration curves to the right illustrate a comparison of the relative protein mobility (RF). Barely any differences are detectable between separation characteristics of electrophoresis in line with the invention as compared to gels according to established PAGE procedures where samples were free of guanidinium.

### Example 2

The effectiveness and performance of the invented gel electrophoresis for direct analysis of proteins samples containing guanidinium-hydrochloride as the chaotropic agent was investigated. The experiments were performed as described in the Example 1, except using 16% polyacrylamide gels. The protein samples from two different bacterial extracts proteins solubilized in 6M guanidinium hydrochloride were separated using the Schaegger system (tris/tricine-PAGE) as illustrated in Fig. 4. Resolution of protein bands and the relative protein mobility after electrophoresis as disclosed herein are well comparable to the separation characteristics of gels according to established PAGE procedures with samples free of guanidinium (TCA precipitates).

### Example 3

The performance of direct analysis of samples containing guanidinium hydrochloride as the chaotropic agent was investigated as described herein. Electrophoresis was performed as described in the Example 1, except for using a tris/glycine system. For the sample buffer used please refer to Fig. 1. 8% polyacrylamide gels were cast with a modified tris-HCl gel buffer as described herein (cf. Fig. 3). A tris/glycine running buffer was used as shown in Fig. 2, containing 0.01% N-Lauroylsarcosine in the cathode buffer. As illustrated in Fig. 5 the protein samples with 6M guanidinium separated under conditions according to the present disclosure, were compared to such protein samples after TCA precipitation separated by SDS-PAGE under standard conditions of the Laemmli method. The resolution of protein bands and the relative protein mobility after electrophoresis are well comparable.

### Example 4

The effectiveness and performance of the invented gel electrophoresis for direct analysis of proteins samples containing guanidinium-hydrochloride as the chaotropic agent was investigated. The tests were performed as described in the Example 3, except using the tris/glycine system with modified 12% polyacrylamide gels as described herein. Fig. 5 illustrates the separation of protein samples solubilized in 6M guanidinium hydrochloride under conditions according to the present disclosure, compared to identical samples after TCA precipitation and separated by tris/glycine SDS-PAGE under standard conditions of the Laemmli method.

### Example 5

The performance of direct analysis of samples containing guanidinium hydrochloride as the chaotropic agent was investigated as described herein. The experiments were essentially performed as described for Example 1. A precast Criterion™ XT Bistris gel system (from Bio-Rad Laboratories GmbH, Muenchen) with 10% polyacrylamide concentration was used. The illustrated gel in Fig. 6 was loaded with samples from bacterial extracts containing solubilized in 6M guanidinium hydrochloride with a N-Lauroylsarcosine-containing sample buffer described in Fig. 1. A tris/MOPS electrophoresis running buffer was used as shown in Fig. 2, containing 0.01% N-Lauroylsarcosine in the cathode buffer. HM and LM indicate standard protein mixtures of high and low molecular mass that were used to define the protein mobility during the electrophoresis.

### Example 6

The effectiveness and performance of the invented gel electrophoresis for direct analysis of proteins samples containing guanidinium-hydrochloride as the chaotropic agent was investigated. The tests were performed as described in the Example 5, except for using a precast Criterion™ XT Bistris gel system (from Bio-Rad Laboratories GmbH, Munich, Germany) with a 4-to-12% gradient polyarylamide concentration. A tris/MOPS electrophoresis running buffer was used as shown in Fig. 2, containing 0.01% N-Lauroylsarcosine in the cathode buffer.

### Example 7

An unmodified 12 % polyacrylamide gel according to the Laemmli method was investigated for direct electrophoretic analysis of samples containing guanidinium hydrochloride as the chaotropic agent. The gel was loaded samples containing defined concentrations of the bacterial protein extracts solubilized in 6M guanidinium hydrochloride with a N-Lauroylsarcosine-containing sample buffer as represented in Fig. 1. A tris/glycine electrophoresis running buffer was used as represented in Fig. 2, containing 0.025% N-Lauroylsarcosine in the cathode buffer. Please note that the depicted tris/glycine PAGE was performed according to the method disclosed herein, but using a standard gel according to Laemmli without modification (gel buffer conditions as given in Fig. 3). For evaluation of the full performance of the method described herein please refer to Example 3 and 4 emphasizing the benefit of the tris/glycine-PAGE with modified gel buffer as described herein.

### Example 8

The performance of gel electrophoresis for direct analysis of proteins samples containing guanidinium hydrochloride as the chaotropic agent was investigated. The tests were essentially performed as described in Example 1. In this example a 10%. Bistris polyacrylamide gel was casted with buffers as given in Fig. 3. For electrophoresis a tris/MOPS running buffer was used as shown in Fig. 2, containing 0.025% N-Lauroylsarcosine in the cathode buffer. Illustrated in Fig. 7 is the separation of samples from different bacterial extracts with 6xHis-tagged fusion proteins solubilized in 6M guanidinium hydrochloride. N-Lauroylsarcosine-containing sample buffer (as specified in Fig. 1) was used under conditions as described herein. The resolution of protein bands and mobility of the loaded standard protein mixtures (indicated as HM and LM) are well comparable to the characteristics of a Bistris gel performed according to established SDS-PAGE procedure when samples without guanidinium were applied (TCA precipitates).

### Example 9

The effectiveness and performance of the invented gel electrophoresis for direct analysis of proteins samples containing guanidinium-hydrochloride as the chaotropic agent was investigated using a 13% Bistris polyacrylamide gel. The tests were performed as described in Example 8 using the N-Lauroylsarcosine-containing sample buffer and running buffer. Results of the electrophoretic separation under conditions as described herein is illustrated in Fig. 7, and compared to such samples after TCA precipitation (without guanidinium) in SDS-containing sample buffer separated under standard SDS-PAGE conditions.

### Example 10

The performance of the N-Lauroylsarcosine-containing sample buffer as described herein was investigated in comparison to standard SDS-sample buffer of Laemmli method (as specified in Fig. 1). Standard protein mixtures (indicated as HM and LM) without guanidinium were loaded using the indicated sample buffer. Electrophoretic separation was performed with tris/glycine PAGE according to the Laemmli method. As illustrated in Fig. 8, the N-Lauroylsarcosine-containing sample buffer performed equally well in the tris/glycine system with comparable mobility of the standard proteins.

### Example 11

The performance of the the N-Lauroylsarcosine-containing sample buffer as described herein was investigated in comparison to standard SDS-sample buffer of Laemmli method (sample buffers are specified in Fig. 1). The tests were essentially performed as described in Example 10. In this example, the electrophoretic separation was performed using tris/tricine PAGE according to the Schaegger method. As shown in Fig. 8, the N-Lauroylsarcosine-containing sample buffer performed equally well with comparable resolution and protein mobility.

### Example 12

The performance of the N-Lauroylsarcosine-containing sample buffer and the influence of N-Lauroylsarcosine in the tris/glycine running buffer as described herein was determined and compared to standard conditions according to the Laemmli method. The buffer compositions are specified in Figures 1, 2 and 3. Standard protein mixtures of high and low molecular mass (HM, LM) were loaded with the indicated sample buffers. Electrophoresis running buffers were used either with or without the addition of N-Lauroylsarcosine and EDTA. Calibration curves of Fig. 9A show the comparison of the relative protein mobilities (RF). The results of buffer conditions with N-Lauroylsarcosine according to the present disclosure are well comparable to standard conditions according to the Laemmli method.

### Example 13

The performance of the N-Lauroylsarcosine-containing sample buffer in comparison to Laemmli SDS-sample buffer and the influence of N-Lauroylsarcosine in a tris/tricine running buffer as described herein was determined. Experiments were essentially performed as described in Example 12. A tris/tricine electrophoresis was performed either with or without the addition of N-Lauroylsarcosine and EDTA. A comparison of the relative protein mobility (RF) is illustrated in the calibration curves of Fig. 9. Barely any differences of the separation characteristics are detectable between the different buffer conditions with and without N-Lauroylsarcosine.

### Example 14

Immunochemical detection of specific recombinant proteins after polyacrylamide gel electrophoresis for direct analysis of proteins samples containing guanidinium hydrochloride as the chaotropic agent, in line with the disclosure of this document. Protein samples from two different bacterial extracts each containing different 6xHis-tagged recombinant fusion proteins (S100A9 or p120) solubilized in 6M guanidinium hydrochloride were used. The samples were separated under denaturing conditions using 5 different gel systems: the Schaegger system (tris/tricine PAGE) with 10% or 16% polyacrylamide gels, the Laemmli method using 8% or 12% polyacrylamide gels and Bistris gels with 10% polyacrylamide concentration. The semi-dry Western Blot and immunodetection (Schnaeker, E.M., et al., Cancer Res. [2004] 64, 24, 8924-8931) was performed after the samples with 6M guanidinium hydrochloride were directly analyzed using the electrophoresis conditions as described herein. Buffer compositions are specified in Figures 1, 2 and 3. As illustrated in Fig. 10, all recombinant proteins could be well detected by specific antibodies demonstrating the performance of the invented PAGE system for the application of subsequent procedures and methods.

### Example 15

Direct analysis of proteins samples containing guanidinium hydrochloride as the chaotropic agent by SDS-PAGE. Electrophoresis was performed with established gel and running buffer conditions according to the Schaegger method (tris/tricine PAGE, 16% polyacrylamide). Fig. 12 shows the results of samples from bacterial extracts in increasing guanidinium concentrations between 0.4 and 6.0 M, loaded with SDS containing sample buffer.

### Example 16

The results of a direct gel electrophoretic analysis of proteins samples containing guanidinium hydrochloride as the chaotropic agent are shown in Fig. 13. Electrophoresis running buffer and gel buffer were used according to the Schaegger method (tris/tricine PAGE, 16% polyacrylamide). Samples from bacterial extracts containing 6xHis-tagged fusion proteins with increasing guanidinium concentrations between 0.4 and 6.0 M were loaded with N-Lauroylsarcosine-containing sample buffer as described herein.

## Claims

1. A method of separating components of a sample by electrophoresis, wherein the sample comprises a guanidinium salt, a potassium salt and/or a chaotropic agent, the method comprising adding to the sample N-Lauroylsarcosine, and carrying out electrophoresis.

2. The method of claim 1, wherein the chaotropic agent is one of guanidinium hydrochloride, guanidinium thiocyanate, sodium thiocyanate and potassium thiocyanate.

3. The method of claims 1 or 2, wherein the sample further comprises a reducing agent.

4. The method of any one of claims 1 to 3, comprising carrying out electrophoresis under denaturing conditions.

5. The method of any one of the preceding claims, wherein the electrophoresis is gel electrophoresis.

6. The method of claim 5, comprising carrying out gel electrophoresis with a cathode running buffer comprising at least one of N-Lauroylsarcosine and a metal chelating agent.

7. The method of claims 5 or 6, wherein the gel electrophoresis is polyacrylamide gel electrophoresis (PAGE) or agarose gel electrophoresis.

8. The method of any one of the preceding claims, wherein the electrophoresis is SDS-PAGE or LDS-PAGE.

9. The method of any one of the preceding claims, comprising adjusting the pH of the sample to a value of 7.5 or higher before carrying out electrophoresis.

10. The method of claim 6, wherein the metal chelating agent comprises one or more of ethylenediaminetetraacetic acid (EDTA), ethylene glycol tetraacetic acid (EGTA), diethylenetriaminepentaacetic acid (DTPA), N diaminocyclohexanetetraacetic acid (DCTA), nitrilotriacetic acid (NTA), 1,2-bis(o-aminophenoxy)ethane-N,N,N',N'-tetraacetic acid (BAPTA), diethyldithiocarbamate (DEDTC), glutamic acid diacetic acid (GLDA) and tripolyphosphate (TPP), 2,3-dimercapto-1-propanol (dimercaprol), porphine and heme.

11. The method of any one of claims 1 - 10, comprising:
a) adding to a protein sample comprising the guanidinium salt, the potassium salt and/or the chaotropic agent, a sample buffer having a pH of at least 8.0, the sample buffer comprising (i) a buffer compound, (ii) a concentration of about 0.2% (w/v) or more of N-Lauroylsarcosine, and (iii) a concentration of about 0.2 mM or more of a reducing agent;
b) applying the sample to an electrophoresis gel; and
d) exposing the electrophoresis gel to an electric field between an anode and a cathode, the anode being encompassed in an anode buffer and the cathode being encompassed in a cathode buffer, wherein the cathode buffer comprises about 0.003% (w/v) or more of N-Lauroylsarcosine.

12. The method of any one of the preceding claims, wherein the sample buffer further comprises a chelating agent.

13. The method of claims 11 or 12, wherein the buffer compound comprises 2-amino-2-hydroxymethyl-propane-1,3-diol (tris), and/or the chelating agent comprises EDTA and/or the reducing agent comprises dithiothreitol (DTT) or 2-mercaptoethanol.

14. The method of any one of the preceding claims, wherein the electrophoresis running buffer further comprises:
(a) about 0.05 M tris, about 0.05 M of 3-(N-morpholino)propanesulfonic acid (MOPS), about 1 mM or more of EDTA, about 0.1% (w/v) SDS, about 0.01 % (w/v) or more of N-Lauroylsarcosine, and about 1 mM NaBisulfite; or
(b) about 0.025 M tris, about 190 mM glycine, about 1.5 mM or more of EDTA, about 0.1 % (w/v) SDS, and about 0.01 % or more (w/v) N-Lauroylsarcosine; or
(c) an anode running buffer comprising about 0.1 M tris, and a cathode running buffer comprising about 0.1 M tris, about 0.1 M N-(2-hydroxy-1,1-bis(hydroxyl-methyl)ethyl)glycine (tricine), about 1.5 mM or more of EDTA, about 0.1 % (w/v) SDS, and 0.01 % (w/v) or more N-Lauroylsarcosine.

15. An electrophoresis sample buffer with a pH value of 8.0 or more, the electrophoresis sample buffer comprising a buffer compound at a concentration of 200 mM or less, N-Lauroylsarcosine, and a reducing agent.

16. The electrophoresis sample buffer of claim 15, wherein the reducing agent comprises one or more of 2-mercaptoethanol, dithiothreitol (DTT), tris(2-carboxyethyl)phosphine (TCEP), dithioerythritol (DTE), reduced glutathione, cysteamine, tri-n-butylphosphine (TBP), dithioerythriol, 2-mercaptoethylamin-HCl, dithiobutylamine (DTBA), cysteine, cysteine-thioglycolate, thioglycolic acid and hydroxyethyldisulphide (HED).

17. The electrophoresis sample buffer of claims 15 or 16, having a pH of about 8.5 and comprising about 0.063 M tris, about 3% (w/v) N-Lauroylsarcosine, about 20% (v/v) glycerol, about 100 mM DTT, and about 3 mM EDTA.

18. The use of a gel buffer for gel electrophoretic analysis of a sample, the sample comprising a guanidinium salt, a potassium salt and/or a chaotropic agent, wherein the gel electrophoretic analysis comprises a tris/glycine electrophoresis system, the gel electrophoresis gel buffer having a pH value of about 8.0 or more and comprising 0.6 M or more of a buffer compound.

19. The use of claim 18, comprising application of the gel buffer to both the stacking gel and the separating gel in a tris/glycine electrophoresis system.

20. The use of a sample buffer for gel electrophoretic analysis of a sample, the sample buffer comprising a buffer compound, N-Lauroylsarcosine, and a reducing agent, wherein the gel electrophoretic analysis comprises a tris/glycine electrophoresis system or a tris/tricine electrophoresis system.

21. A gel electrophoresis running buffer, the gel electrophoresis running buffer having a pH value of about 6.7 or more, the gel electrophoresis running buffer comprising a buffer compound, about 0.1 % (w/v) or more of a detergent, and about 0.003 % (w/v) or more of N-Lauroylsarcosine.

22. The electrophoresis sample buffer of any one of claims 15 to 17, or the gel electrophoresis running buffer of claim 21, wherein the buffer compound comprises tris.

23. A kit for the separation of components of a sample by gel electrophoresis, the sample comprising a guanidinium salt, a potassium salt and/or a chaotropic agent, wherein the kit comprises an electrophoresis sample buffer, and a gel electrophoresis running buffer, the electrophoresis sample buffer comprising a buffer compound, N-Lauroylsarcosine, and a reducing agent, and the gel electrophoresis running buffer comprising N-Lauroylsarcosine.

24. The kit of claim 23, further comprising a tris/glycine gel electrophoresis gel buffer, wherein the gel electrophoresis gel buffer has a pH value of about 7.0 or more, the gel electrophoresis gel buffer comprising about 0.6 M of a buffer compound or more, and about 0.1 % (w/v) of a detergent or more.

25. Use of (i) an electrophoresis sample buffer, comprising a buffer compound, N-Lauroylsarcosine, and a reducing agent, (ii) a electrophoresis running buffer, having a pH value of about 7.0 or more, and comprising a buffer compound and about 0.01 % (w/v) or more of N-Lauroylsarcosine, (iii) the gel electrophoresis running buffer of claim 21, and/or (iv) the kit of claims 23 or 24 for the electrophoretic analysis of a sample, wherein the sample comprises a guanidinium salt, a potassium salt and/or a chaotropic agent.

26. The use of claim 25, wherein the electrophoretic analysis is denaturing gel electrophoresis.
